(19) Europäisches Patentamt
European Patent Office
Office européen des brevets

(11) Veröffentlichungsnummer: **0 433 526 A1**

(12) # EUROPÄISCHE PATENTANMELDUNG

(21) Anmeldenummer: **89890328.1**

(51) Int. Cl.5: **C07C 281/18**, A61K 31/155

(22) Anmeldetag: **19.12.89**

(43) Veröffentlichungstag der Anmeldung: **26.06.91 Patentblatt 91/26**

(84) Benannte Vertragsstaaten: **BE CH DE ES FR GB IT LI NL SE**

(71) Anmelder: **ARZNEIMITTELWERK DRESDEN GMBH**
**Wilhelm-Pieck-Strasse 35**
**O-8122 Radebeul(DE)**

(72) Erfinder: **Richter, Peter, Prof. Dr.sc.nat.**
**Makarenkostrasse 14 A**
**O-2200 Greifswald(DE)**
Erfinder: **Hagen, Angela, Dr.rer.nat.**
**Kühlungsborner Strasse 29**
**O-1093 Berlin(DE)**
Erfinder: **Grünheid, Kristine, Biol.-Ing.**
**Paul-Dessau-Strasse 9**
**O-1142 Berlin(DE)**
Erfinder: **Besch, Anita, Dr.rer.nat.**
**Makarenkostrasse 26 A**
**O-2200 Greifswald(DE)**
Erfinder: **Schleuder, Manfred, Dr.rer.nat.**
**Wilhelm-Pieck-Allee 122**
**O-2200 Greifswald(DE)**
Erfinder: **Albrecht, Detlev, Dr.rer.nat.**
**Weststrasse 6**
**O-8029 Dresden(DE)**
Erfinder: **Kasbohm, Katharina, Dipl.-Pharm.**
**Johann-Sebastian-Bach-Strasse 33**
**O-2200 Greifswald(DE)**

(74) Vertreter: **Puchberger, Rolf, Dipl. Ing. et al**
**Patentanwälte, Dipl. Ing. Georg Puchberger Dipl. Ing. Rolf Puchberger Dipl. Ing. Peter Puchberger Singerstrasse 13 Postfach 55**
**A-1010 Wien(AT)**

(54) **Mittel zur Behandlung von Herz-Kreislauf-Erkrankungen, insbesondere von Arrythmien.**

(57) Die Erfindung betrifft Mittel zur Behandlung von Herz-Kreislauf-Erkrankungen, insbesondere von Arrhythmien, gekennzeichnet durch einen Gehalt an Salzen substituierter 2-Aminobenzophenonamidinohydrazone, die als Z- oder E-Isomere oder als Isomerengemische vorliegen können und mit an sich üblichen pharmazeutischen Hilfsstoffen, wie Trägerstoffen, Gleitmitteln, Isotonisierungszusätzen oder Antioxidantien zu pharmazeutischen Zubereitungen, wie Tabletten, Dragees, Tropf-oder Injektionslösungen verarbeitet werden.
Erfindungsgemäß werden die Salze substituierter 2-Aminobenzophenonamidinohydrazone mit antiarrhythmischer und antiischaemischer Wirkung erhalten, indem substituierte 2-Aminobenzophenone mit Hydrazin-Derivaten kondensiert und die Zwischenprodukte auf verschiedenen Wegen in Amidinohydrazonsalze überführt werden, aus denen nach Trennung der Isomeren oder Isomerisierung die zugrundeliegenden Basen freigesetzt und mit Säuren umgesetzt werden.
Anwendungsgebiet der Erfindung ist die pharmazeutische Industrie.



Xerox Copy Centre

# MITTEL ZUR BEHANDLUNG VON HERZ-KREISLAUF-ERKRANKUNGEN, INSBESONDERE VON ARRHYTHMIEN

Die Erfindung betrifft Mittel zur Behandlung von Herz-Kreislauf-Erkrankungen, insbesondere von Arrhythmien, gekennzeichnet durch einen Gehalt einer Verbindung der allgemeinen Formel 1 als Wirkstoff, in der

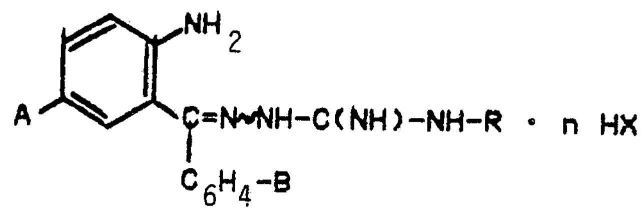 1

A = H, Halogen, $NH_2$.HX, Alkyl, $NO_2$,
B = H, Halogen, OH, Alkyl,
R = H, Phenyl, subst.Phenyl,
n = 0, 1, 2
HX = eine physiologisch verträgliche Mineralsäure, Alkansulfonsäure, Alkansäure, Alkensäure, Hydroxyalkansäure, Aminoalkansäure, Alkandisäure, Hydroxyalkandisäure bedeuten und die als 2- oder E-Isomere oder als Isomerengemische vorliegen können.

Die Erfindung ist in der pharmazeutischen Industrie anwendbar.

Unter Amidinohydrazonen aromatischer Ketone wurden zahlreiche biologisch aktive Vertreter aufgefunden.

So wurden die Amidinohydrazone des Benzophenons und des 4-Hydroxybenzophenons als analgetisch, spasmolytisch und antiinflammatorisch wirksam beschrieben (Ugine Kuhlman, Fr.P.M. 7.858, Fr. 181 927, vom 30.12.1968, ausg. 20.04.1970, Kl. A 61 k, C 07 c).

Antifungale, antibakterielle, antivirale und Antimycoplasmawirkungen sollen N-substituierte Amidinohydrazone 3,4,4′-trisubstituierter Ben zophenone aufweisen, die im Benzophenonrest Cl, $CH_3$, 0H und $0CH_3$ als Substituenten tragen (Nishimura, T., et al., Jap.P. 79 115 330, vom 27.02.1978, ausg. 07.09.1979; Kl. A 61 k 31/15, C 07 c 133/10).

Besonders intensiv wurden mehrfachsubstituierte Benzophenonamidinohydrazone bearbeitet, die in der Regel Halogen bzw. Trifluormethylsubstituenten in 3- und 4- bzw. 4′-Position tragen. Diese Verbindungen wurden vor allem wegen ihrer Eignung als Antimalariamittel (Do Amaral, J.R., et al., J. med. Chem. 12, 21 (1969); Ruiz, R., und D.M. Aviado, Pharmacology 4, 45 (1970); French, F.A., et al., J.med. Chem. 14, 862 (1971) sowie wegen ihrer antineoplastischen (cytotoxischen) Eigenschaften (Baiocchi, F., et al. J. med. Chem. 6, 431 (1963); French, F.A., et al., Cancer Chemother. Rep. part 2, 2, 177 (1977) dargestellt.

Bei zwei der erwähnten Verbindungen mit Antimalariaaktivität wurde als Nebenwirkung eine schwache antiarrhythmische Wirkung beschrieben. Es handelt sich dabei um das 4-Fluor-4′-trifluormethylbenzophenonamidinohydrazon und das 3,4-Dichlor-4′-trifluormethylbenzophenonamidinohydrazon. Wegen der geringen Stärke dieser Wirkqualität, sowie der infolge hoher Toxizität sehr geringen therapeutischen Breite sind diese Verbindungen jedoch nicht geeignet für eine therapeutische Nutzung (Ruiz, R., und D.M. Aviado, a.a.O.).

Für Amidinohydrazone mehrfach substituierter Benzophenone mit Br, Cl und $CH_3O$ als Substituenten in 3- und/oder 4-Stellung wurde eine Hemmwirkung gegenüber dem Angiotensin-Converting-Enzym (ACE) beschrieben (Kühmstedt, H., Wunderlich, I., Gräfe, I., Heder, G., Siems, E., und Piske, K., DD-WP 229 398 A1, v. 05.12.1984, ausg. 6. 11. 85, Kl. C 07 c 133/12, A 61 k 31/155), wobei es sich meist um Hydriodide oder um Nitrate der Titelverbindungen, also um Salze von Säuren, handelt, die für Pharmazeutika eher seltener verwendet werden.

Unter den durch den Erfindungsanspruch dieser Patentschrift erfaßten Verbindungen finden sich keine, die der allgemeinen Formel 1 genügen. Allerdings werden in einer tabellarischen Übersicht Angaben über die ACE-Hemmwirkung einiger der Formel 1 entsprechenden 2-Aminobenzophenonamidinohydrazone gemacht. Da jedoch ein ursächlicher Zusammenhang von ACE-Hemmwirkung und antiarrhythmischer Wirksamkeit weder bekannt ist, noch unter Anwendung des Fachwissens von Sachkundigen abgeleitet werden kann, wird dadurch die Neuheit der Mittel zur Behandlung von Arrhythmien, gekennzeichnet durch einen Gehalt einer

Verbindung der allgemeinen Formel 1, nicht beeinträchtigt.
Außerdem sind die erwähnten Salze wegen zu geringer Löslichkeit und ungenügender Standardisierbarkeit für die Herstellung pharmazeutischer Zubereitungen ungeeignet. Die Eigenschaften einiger für die Salzbildung angegebener Säuren bedingen die Instabilität entsprechender Salze.
Darüberhinaus werden sowohl die Herstellung als auch die physikochemischen Eigenschaften dieser 2-Aminobenzophenonamidinohydrazone weder beschrieben noch belegt.

Umsetzungen von Carbonylverbindungen mit Hydrazinderivaten und die Überführung der entsprechenden Kondensationsprodukte in Amidinohydrazone sind im Prinzip bekannt (American Cyanamid Co., US.P. 794 600, v. 06.05.1977, ausg. 15. 08. 1978, Kl. A 61 k 31/15, C 07 c 121/60, C 07 c 133/10; S.Schutz, H.-H. Meyer, K. Stoepel und H.G. Kroneberg, GB-PS 1 036 987, v. 29.04.1965, ausg. 20.07.1966, Kl. C 07 c 133/10, C 07 d 5/16, C 07 d 27/00, C 07 d 49/34) bzw. können analog den Verfahren zur Darstellung substituierter Aminoguanidine (s. Houben-Weyl), "Methoden der Organischen Chemie", Georg Thieme Verlag Stuttgart, Bd. 7/1, S. 461 ff. (1954), Bd.8, S. 191 ff. (1952) ) durchgeführt werden.
Gleiches gilt für die Umsetzung von Carbonylverbindungen mit Aminoguanidinen zu Amidinohydrazonen, die sowohl in den o.g.Patentschriften über biologisch aktive Amidinohydrazone sowie in Standardwerken beschrieben ist (s. Houben-Weyl, "Methoden der Organischen Chemie", Georg Thieme Verlag Stuttgart, Bd. 7/1, S. 470 (1954); Bd. 7/2b, S.1958 (1976); Bd. 8/1, S. 192-193 (1952) ).

Bekannt ist gleichfalls, daß Amidinohydrazone unsymmetrischer Ketone jeweils in der Z- oder E-Form auftreten können. Die bisher von ande ren Autoren beschriebenen Amidinohydrazone, bei denen Z-/E-Isomerie möglich ist, sind jedoch meist nicht in die Isomere aufgetrennt bzw. diese ineinander umgewandelt worden.
Auch Kühmstedt et al. (DD-WP 229 398 A1, v. 05.12.1984, ausg.06.11.1985, Kl. C 07 c 133/12, A 61 k 31/155) lassen die Möglichkeit des Auftretens von Z- und E-Isomeren außer Acht und beschreiben keine sterisch einheitlichen Verbindungen.
Dagegen konnten H.King und J.Wright (J.chem. Soc. (London) 1948, 2314) von Isatin-β-amidinohydrazon, das als potentielles Malariamittel bezeichnet wurde, beide Stereoisomere darstellen. Bei der Kondensation von Isatin mit Aminoguanidin in siedendem Eisessig wurde zunächst die Z-Form erhalten, von der das entsprechende Hydrochlorid und Nitrat hergestellt werden konnten. Wenn das Hydrochlorid bei 80 °C im Überschuß 0,5 m wäßriger Natriumhydroxidlösung gelöst wird, wandelt es sich in das E-Isatin-ß-amidinohydrazon um, dessen Hydrochlorid bei Erhitzen in 16% Salzsäure wieder in die Z-Form übergeht.

Während die Darstellung sterisch einheitlicher Verbindungen der allgemeinen Formel 1 bisher nicht beschrieben ist, wurden Oxime, Hydrazone und Ketimine von 2-Aminobenzophenonen dargestellt und die Z- und E-Isomere aufgrund unterschiedlicher Löslichkeit durch fraktionierte Kristallisation oder durch chromatographische Methoden voneinander getrennt (Sternbach, H.L., S.Kaiser und E. Reeder, J. Amer. chem. Soc. 82, 475 (1960); Orzalesi, G., R.Selleri, R. Landi Vittory, F. Innocenti und G. Grandolini, J. heterocycl. Chem. 14, 733 (1977); Bell, S.C., G.L. Conklin und S.J. Childress, J. org. Chemistry 29, 2368 (1964) ).

Der Erfindung liegt die Aufgabe zugrunde, Mittel zur Behandlung von Herz-Kreislauf-Erkrankungen, insbesondere von Arrhythmien, zu entwickeln. Die Aufgabe wurde dadurch gelöst, daß Verbindungen der allgemeinen Formel 1, in der

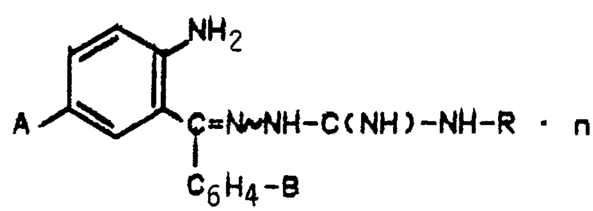 1

A = H, Halogen, $NH_2$.HX, Alkyl, $NO_2$,
B = H, Halogen, OH, Alkyl,
R = H, Phenyl, subst. Phenyl,
n = 0, 1, 2,
HX = eine physiologisch verträgliche Mineralsäure, Alkansulfonsäure, Alkansäure, Alkensäure, Hydroxyalkansäure, Aminoalkansäure, Alkandisäure, Hydroxyalkandisäure bedeuten und die als Z- oder E-Isomere oder als Isomerengemische vorliegen können, auf ihre antiarrhythmische Wirkqualität gegenüber zahlreichen Arrhythmieformen geprüft wurden.

Die Prüfung erfolgt nach intravenöser und oraler Verabreichung an den tierexperimentellen Modellen Aconitinarrhythmie, g-Strophantinarrhythmie und $CA^{2+}$-Arrhythmie sowie gegenüber ischämisch induzierten Arrhythmien am Okklusionsmodell.
Es wurden Verbindungen der allgemeinen Formel 1 mit starker und dosisabhängig auftretender antiaarhythmischer und myokardprotektiver Aktivität gefunden.
Von diesen erwiesen sich (Z)- und (E)-2-Amino-5-chlorbenzophenonamidinohydrazonacetat (Beispiel 21 und 32) als besonders auffällig. Beide Substanzen sind qualitativ annähernd übereinstimmend an differenten experimentellen Modellen antiarrhythmisch wirksam, wobei zusätzliche Vorteile in der geringen intravenösen und peroralen Toxizität an Ratte und Maus, einem hohen therapeutischen Index sowie der relativ langen Wirkungsdauer nach einmaliger Verabfolgung am Aconitinmodell der Ratte gesehen werden.
Beim quantitativen Vergleich der Dosiswirkungskurven war das Z-Isomer etwas wirksamer als das E-Isomer. Ein grundsätzlich unterschiedliches Wirkprofil beider Isomere ist auszuschließen. Die klinische Prüfung in Phase 1 erfolgte mit der Z-Form.
Der neuartige Wirkungsmechanismus sowie die zusätzlichen myokardprotektiven und spasmolytischen Eigenschaften dieser Substanzen sowie weiterer Verbindungen der allgemeinen Formel I belegen, daß die Erfindung in der pharmazeutischen Industrie umfassend anwendbar ist.

Zusammenfassend läßt sich das (Z)-2-Aminos-chlorbenzophenonamidinohydrazonacetat (Beispiel 21) folgendermaßen einschätzen:
- Die Verbindung entfaltet eine protektive Wirkung sowohl gegenüber chemisch induzierten Arrhythmien als auch gegenüber den nach Koronaarterienligatur ausgelösten arrhythmogenen Störungen, für die wahrscheinlich die größte Übereinstimmung zum pathophysiologischen Entstehungsmechanismus der am kranken menschlichen Herzen auftretenden Arrhythmien erwartet werden kann.
- Eine Gegenüberstellung der die antiarrhythmischen Wirkungsstärke charakterisierenden, quantitativen Kenngrößen soll die hohe Wirkativität des neuen, potentiellen Antiarrhythmikums hervorheben. So erwies sich die Substanz beispielsweise am Aconitinmodell der Ratte nach i.v.- und p.o. Gabe im Vergleich zu Chinidin, Ajmalin, Disopyramid und Procainamid deutlich überlegen. Die am gleichen Modell geprüften Verbindungen Lidocain, Mexiletin und Diphenylhydantoin entfalteten i.v. erst in Dosierungen eine Wirkung, bei denen eine Überlagerung pharmakodynamischer und toxischer Effekte offensichtlich war. Dieselben Substanzen waren p.o. der neuen Verbindung weiter unterlegen.
Auch die ß-Rezeptorenblocker Propranolol und Talinolol zeigten eine wesentlich geringere Hemmaktivität an der Aconitinarrhythmie. Annähernd übereinstimmende Aktivität ergab sich zu Falirythmin und Etmozin.
- In Analogie zum Aconitinmodell werden die in der Frühphase nach Okklusion ausgelösten Arrhythmien durch (Z)-2-Amino-5-chlorbenzophenonamidinohydrazonacetat wirkungsvoller antagonisiert als durch Chinidin, Procainamid, Disopyramid, Lidocain, Mexiletin und Propranolol.
- An der g-Strophantinarrhythmie des Meerschweinchens konnten deutliche Wirkungsunterschiede zu Chinidin, Propranolol und Falirytmin herausgearbeitet werden, deren Wirkungsstärke nicht die der neuen Verbindung bei prophylaktischer intravenöser Verabreichung erreichten.
- Auffallend ist in Akutversuch an der Ratte der ermittelte hohe therapeutische Index ($Q = LD_{50}/ED_{20}$) des neuen Antiarrhythmikums im Vergleich zu verschiedenen Therapeutika bei intravenöser Injektion von 40,0 gegenüber Chinidin (5,1), Ajmalin (6,8), Procainamid (3,1), Propranolol (3,4), Etmozin (1,8), Tachmalcor-(25,5)und Falirytmin (11,5), der sich auch nach einmaliger oraler Applikation zu 74,3 (vgl. Chinidin : $Q_{p.o.}$ = 4,9) berechnen ließ.
Unter Berücksichtigung aller toxikologischen Befunde und der jeweiligen Versuchsbedingungen (Mehrfachapplikation) wurden für die neue Verbindung Dosen von 25 mg/kg an der Ratte bzw. 30 mg/kg am Minischwein per os als gut verträglich eingeschätzt.
In den höchsten Prüfdosen (50 und 100 mg/kg) begannen an Ratten toxische Kreislaufsymptome, wie sie auch für andere antiarrhythmika bekannt bzw. zu erwarten sind.
Am Minischwein konnte eine morphologisch faßbare Schädigung ausgeschlossen werden.
- Elektrophysiologische Untersuchungen bestätigen ganz klar die potenten, antiarrhythmischen Eigenschaften der Substanz, die eine ausgeprägte Verzögerung der elektrischen Wiedererregbarkeit am normalen Aktionspotential (AP) isolierter, rechtsventrikulärer Papillarmuskel des Meerschweinchens bewirkte.
Als besonders eindrucksvoll wurde die Verminderung der maximalen Depolarisationsgeschwindigkeit $Na^+$-vermittelter Aktionspotentiale bezeichnet. Auffallend ist in fast allen Versuchen die nachhaltige, mit steigender Inkubationszeit der Präparate zunehmender Wirkung der Substanz. Insgesamt wurde aus den Untersuchungen am Papillarmuskel anhand der Verzögerung der AP-Restitution sowie der Verkürzung der AP-Dauer eine Einordnung der neuen antiarrhythmisch wirksamen Verbindung in die Klasse Ib nach VAUGHAN WILLIAMS abgeleitet. Eine Hemmung des schnellen $Na^+$-Einwärtsstromes konnte ebenfalls an isolierten, adulten Rattenherzzellen mittels voltage-clamp-Technik festgestellt werden, womit Klassifizierungsmerkmale

der Gruppe I gegeben sind.

- Abschließend läßt sich feststellen, daß das pharmakodynamische Wirkprofil des (Z)-2-Amino-5-chlorbenzophenonamidinohydrazonacetats als neuartig zu bezeichnen ist.

Die Substanz wirkt bei i.v.- und p.o.-Applikation dosisabhängig und langanhaltend antiarrhythmisch. Sie besitzt eindeutige Klasse-I-Merkmale nach VAUGHAN WILLIAMS, ohne eine lokalanästhetische Wirkkomponente aufzuweisen. Auch die herausragende Arrhythmiehemmung gegenüber Aconitin könnte als Bestätigung für diesen Aspekt des Wirkungsmechanismus gewertet werden, da der molekulare Mechanismus von Aconitin auf einer Stimulation des $Na^+$-Einstromes über den schnellen potentialabhängigen $Na^+$-Kanal beruhen soll.

Die Substanz entfaltet keine spezifischen α- bzw. ß-rezeptorenblokkierenden Eigenschaften.

Die vorhandene,deutlich antiserotonlnerge Wirkaktivität am Rattenuterus ist nicht als spezifische $5\text{-}HT_1$-Blockade zu werten, sondern vermutlich auf die nachgewiesene spasmolytische Wirkung der Substanz zurückzuführen. Der für die meisten therapeutisch eingesetzten Antiarrhythmika vorhandene, negativ inotrope Effekt ist auch hier zu beobachten, wobei dieser allerdings in vergleichsweise hohen Wirkkonzentrationen in Erscheinung trat und teilweise einen protrahierten Verlauf erkennen ließ. Als weiterer zusätzlicher Vorteil der Substanz ist ihre myokardprotektive Wirkqualität zu werten.

- Auf Basis der vorliegenden pharmakologischen und toxikologischen Befunde wurde die klinische Prüfung des neuen potentiellen Antiarrhythmikums in Stufe I am Menschen absolviert. Dabei wurden klinisch-elektrophysiologische Wirkungen, hämodynamische Effekte sowie die Verträglichkeit bei intravenöser Applikation beurteilt. Im Ergebnis dieses Untersuchungsprogrammes am Menschen wurde aufgrund der nachgewiesenen antiarrhythmischen Potenzen sowie elektrophysiologischen Effekte der neuen Substanz - bevorzugt auf Kammerebene - die Empfehlung zur Testung in Stufe II der klinischen Erprobung ausgesprochen. Die antiarrhythmische Aktivität der Verbindung beginnt beim Menschen bei 0,06 mg/kg und erscheint ausreichend in einer Dosierung ab 0,25 mg/kg.

Für die Anwendung als Mittel zur Behandlung von Arrhythmien werden die Verbindungen der allgemeinen Formel 1 mit an sich üblichen pharmazeutischen Hilfsstoffen, wie Trägerstoffen, Gleitmitteln, Isotonisierungszusätzen oder Antioxidantien zu pharmazeutischen Zubereitungen, wie Tabletten, Dragees, Tropflösungen oder Injektionslösungen, verarbeitet. Diese enthalten pro Dosierungseinheit 2 bis 100 mg, vorzugsweise 15 bis 30 mg, einer zu mindestens 90% als Z-Isomeres vorliegende Verbindungen der allgemeinen Formel 1.

Zur peroralen Anwendung als Antiarrhythmika werden die Verbindungen der allgemeinen Formel 1, vorzugsweise das (Z)-2-Amino-5-chlorbenzophenonamidinohydrazonacetat, unter Zusatz von Trägerstoffen und Gleitmitteln ohne Granulierung zu Tabletten verpreßt, die ggf. in üblicher Weise dragiert werden. Für die parenterale Anwendung werden die Verbindungen der allgemeinen Formel 1, vorzugsweise das (Z)-2-Amino-5-chlorbenzophenonamidinohydrazonacetat, in Wasser gelöst, durch Sterilfiltration keimfrei gemacht und die in Braunglas-Ampullen abgefüllte Lösung vor Anwendung mit Isotonisierungszusätzen versehen oder unter Zusatz von Antioxidantien in verd. 1,2-Propylenglykol gelöst und sterilfiltriert oder die unter Verwendung von Zuckeralkoholen isotonisierten wäßrigen Lösungen nach Sterilfiltration in Braunglas-Kapsolutfläschchen der Lyophilisation unterworfen und sogleich verschlossen.

Die Ampullen bzw. Kapsolutfläschchen werden unter Lichtschutz aufbewahrt.

Die Verbindungen der allgemeinen Formel 1 werden dargestellt, indem 2-Aminobenzophenone der allgemeinen Formel 2, in der

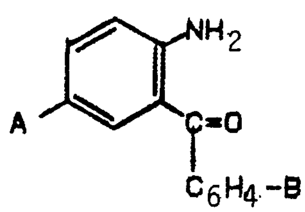

2

A = H, Halogen, $NH_2$. HX, Alkyl, $NO_2$,

B = H, Halogen, OH, Alkyl bedeuten, mit Hydrazinderivaten der Formel 3, in der

$H_2N\text{-}NH\text{-}D$ 3

D = H, CS-NH-R, CSS-Alkyl oder C(NH)-NH-R sowie

R = H, Phenyl oder substituiertes Phenyl bedeuten,

zu Kondensationsprodukten der allgemeinen Formeln 4 bzw. 5 umgesetzt werden, in denen

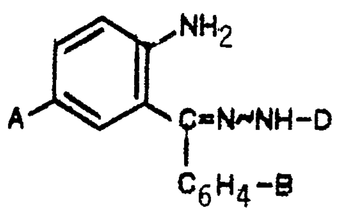

4

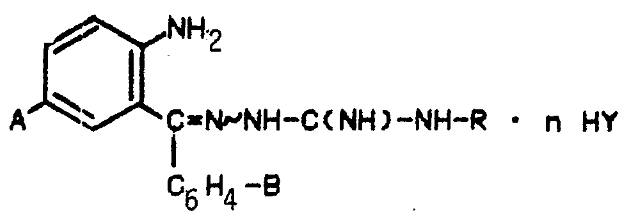

5: n = 1, 2

6: n = 0

A = H, Halogen, $NH_2$.HX, Alkyl, $NO_2$,
B = H, Halogen, 0H, Alkyl,
R = H, Phenyl, subst. Phenyl,
sowie HY eine Mineral- oder Sulfonsäure bedeuten,
und die Kondensationsprodukte 4 anschließend entweder durch Reaktion mit 1-Amidinopyrazolen bzw. Phenylcyanamiden (D = H) oder durch Reaktion mit Iodalkanen und anschließendem Erhitzen mit Ammoniak (D = CS-NH-R, CSS-Alkyl) und die 5 durch Behandeln mit Natriumhydroxidlösung in die der allgemeinen Formel 1 zugrundeliegenden Basen 6 übergeführt werden.
Die Kondensation der Verbindungen 2 und 3 wird in einem geeigneten Lösungsmittel, vorzugsweise Ethanol oder wäßriges Ethanol, unter Zusatz von Säuren, z.B. Salzsäure oder Toluen-4-sulfonsäure, in der Regel unter Rückflußbedingungen realisiert.
Die Zwischenprodukte der allgemeinen Formel 4, in der D die o.g. Bedeutungen hat, können als Z- bzw. E-Isomere vorliegen, werden jedoch für die weiteren Umsetzungen eingesetzt, ohne sie sterochemisch zu charakterisieren, weshalb auch bei ihren chemischen Bezeichnungen auf entsprechende Angaben verzichtet wird.
Die Reaktion der unsubstituierten Verbindungen 4 (D = H) mit 1-Amidinopyrazolen bzw. Phenylcyanamiden wird in einem geeigneten Lösungsmittel, vorzugsweise Ethanol, durch Erhitzen unter Rückfluß durchgeführt.
Die Umsetzung von Verbindungen 4 mit D = CS-NH-R bzw. CSS-Alkyl und Iodalkanen erfolgt in einem geeigneten Lösungsmittel, vorzugsweise in cyclischen Ethern (z.B. Dioxan, Tetrahydrofuran), bei Raumtemperatur. Nach Beendigung der Reaktion, die ggf. dc kontrolliert wird, werden die Basen der Titelverbindungen durch Erhitzen der mit Ammoniak bzw. mit Aminen versetzten Lösungen auf dem Wasserbad gebildet.
Die Freisetzung der Basen 6 aus den Kondensationsprodukten 5 erfolgt durch Versetzen einer ständig gerührten Suspension der 5 mit Alkalihydroxidlösung, vorzugsweise Natriumhydroxidlösung, bei Raumtemperatur.

Zur Überführung in die Titelverbindungen 1 werden die Basen 6 in einem geeigneten Lösungsmittel, vorzugsweise niedere Alkohole oder Aceton gelöst, die Lösung mit der äquimolaren Menge einer Säure HX versetzt und die Salze erforderlichenfalls durch Zusatz von Ether zur Kristallisation gebracht.

Die Kondensationsprodukte 4 und 5 können jeweils als Z- oder E-Isomere auftreten, wobei in der Regel Gemische der beiden Stereoisomere erhalten werden.

Eine Auftrennung der 5-Isomere kann erforderlichenfalls durch fraktionierte Kristallisation oder Umfällung aus niederen Alkoholen vorgenommen werden, da die Isomerisierung der reinen kristallinen Salze bei Raumtemperatur und nur kurzzeitiger Lichteinwirkung wegen der relativ hohen Energiebarriere zwischen den beiden stereoisomeren Formen nur sehr langsam erfolgt.

Reine E-Isomere der Verbindungen 5 können in einigen Fällen erhalten werden, indem die Kondensation der Verbindungen 2 mit Aminoguanidinhydrochlorid (3, D = C(NH)-$NH_2$.HCl) in einem Gemisch von Propan-2-ol und konz. Salzsäure bei Gegenwart von Formaldehyd oder N,N′-Bis-(2-benzoyl-4-chlorphenyl)-diaminomethan bei Raumtemperatur oder durch kurzzeitiges Erhitzen durchgeführt wird.

Reine Z-Isomere der Verbindungen 5 können erhalten werden, indem die E-Isomere oder Isomerengemische in einem aprotischen Lösungsmittel unter Zusatz von Mineral- oder Sulfonsäuren erhitzt werden, oder indem sie in einem inerten Lösungsmittel mit Acetanhydrid umgesetzt und die entstandenen (Z)-2-Acetaminobenzophenonamidinohydrazone der allgemeinen Formel 7

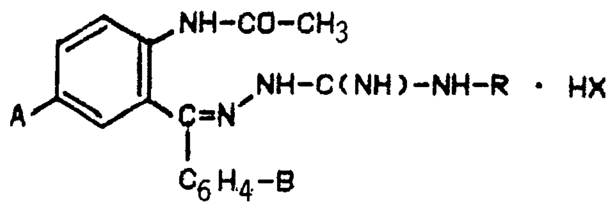

7

in einem Ethanol-Salzsäure-Gemisch entacetyliert werden, oder indem sie in Ethanol oder Aceton in Konzentrationen von 1 bis 10% gelöst und mit Licht der Wellenlänge eines für die E-Isomeren typischen, vorzugsweise zwischen 360 und 380 nm liegenden Absorptionsmaximums bestrahlt werden.

Als Strahlungsquellen kommen entweder Punktstrahler, wie Quecksilber- bzw. Xenon-Höchstdruckstrahler in Kombination mit Steilkantenfiltern, die das gesamte Licht mit Wellenlängen unter 350 nm absorbieren, oder bevorzugt bei 365 nm emittierende Quecksilberhochdruckstrahler in Kombination mit Schwarzglasfiltern in Frage.

Als Festsubstanzen können sowohl die Z-Isomere als auch die E-Isomere der 1 in reiner Form erhalten werden. In Lösungen stellt sich dagegen jeweils ein Gleichgewicht der Stereoisomeren ein, dessen Lage vom Lösungsmittel abhängt und durch Belichtung beeinflußt wird, so daß die Lösungen vor Licht geschützt werden müssen.

Das Vorliegen der kristallinen, substituierten 2-Aminobenzophenonamidinohydrazonsalze in der Z- bzw. E-Form kann durch Röntgenstrukturanalyse bewiesen werden.

So wurden durch Einkristallstrukturanalyse die Molekül- und Kristallstrukturen der aus Ethanol/$H_2O$ kristallisierten pseudopolymorphen Form des (Z)-2-Amino-5-chlorbenzophenonamidinohydrazonacetats.$C_2H_5OH.H_2O$ und des (E)-2-Amino-5-chlorbenzophenonamidinohydrazonacetats bestimmt.

Die kristallografische Charakterisierung erfolgte mit Hilfe von Pulverdiffraktionsuntersuchungen und durch Bestimmung der Gitterparameter auf einem Vierkreisdiffraktometer. Folgende Kristalldaten wurden ermittelt:

(Z) -2-Amino-5-chlorbenzophenonamidinohydrazonacetat. $C_2H_5OH$ . $H_2O$ $C_{16}H_{15}ClN_5O_2.C_2H_5OH.H_2O$, monoklin $P2_1/c$, a = 9,993(1), b = 9,480(2), c = 23,189(3) A, ß = 94,97(1)°, Z = 4, $D_c$ = 1,250 g/$cm^3$, MoKα-Strahlung, 3447 Reflexe, R = 0,068

(Z)-2-Amino-5-chlorbenzophenonamidinohydrazonacetat

$C_{16}H_{15}ClN_5O_2$, tetragonal $P4_12_12_1$ bzw. $P4_32_11$, a = 8,504(8), b = 8,504(8), c = 50,094(46) A, Z = 8, $D_c$ = 1,340 g/$cm^3$

(E )-2-Amino-5-chlorbenzophenonamidinohydrazonacetat

$C_{16}H_{15}ClN_5O_2$ monoklin $P2_1/n$, a = 15,159(1), b = 6,704(1), c = 18,045(3) A, ß = 109,89(2)°, Z = 4, $D_c$ = 1,340 g/$cm^3$, MoKα-Strahlung, 3015 Reflexe, R = 0,073

Tabelle 1 enthält die Atomkoordinaten der Nichtwasserstoffatome von (Z)-2-Amino-5-chlorbenzophenonamidinohydrazonacetat.$C_2H_5OH.H_2O$ und (E)-2-Amino-5-chlorbenzophenonamidinohydrazonacetat Durch das Pulverdiagramm in Tabelle 2 wird die kristalline Phase von (Z)-2-Amino-5-chlorbenzophenonamidinohydrazonacetat identifiziert.

Wie aus den Abbildungen 1 und 2 zu erkennen ist, wurde die sterische Zuordnung für (Z)-2-Amino-5-chlorbenzophenonamidinohydrazonacetat.$C_2H_5OH$ .$H_2O$ und für (E)-2-Amino-5-chlorbenzophenonamidinohydrazonacetat bestätigt, wobei den Abbildungen zugleich die Bindungsabstände und Bindungswinkel entnommen werden können.

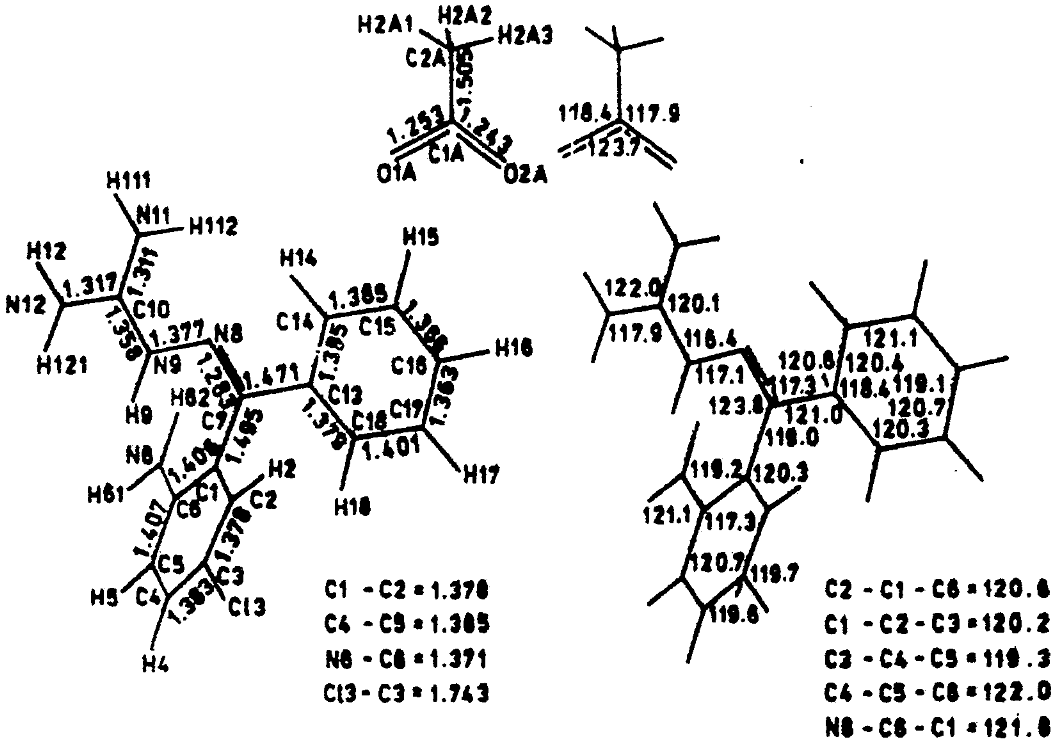

C1 - C2 = 1.378
C4 - C5 = 1.385
N6 - C6 = 1.371
C13 - C3 = 1.743

C2 - C1 - C6 = 120.6
C1 - C2 - C3 = 120.2
C3 - C4 - C5 = 119.3
C4 - C5 - C6 = 122.0
N6 - C6 - C1 = 121.8

Abbildung 1:
Bindungsabstände und Bindungswinkel in (Z)-2-Amino-5-chlorbenzophenonamidinohydrazonacetat.$C_2H_5OH.H_2O$

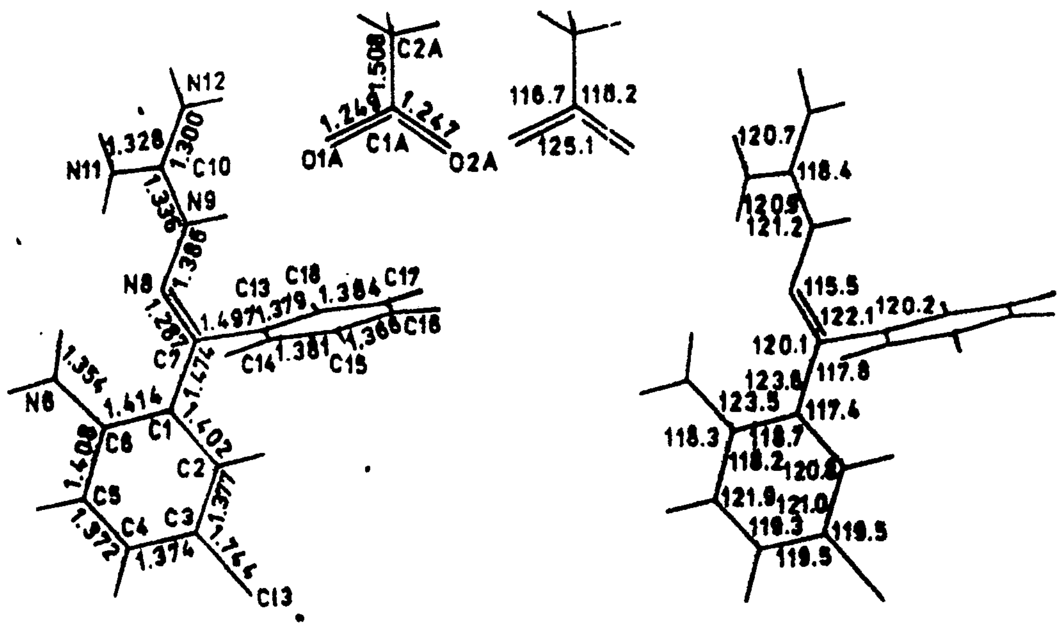

C13 - C14 = 1.388
C18 - C17 = 1.379

C7 - C13 - C14 = 120.8
C14 - C13 - C18 = 119.2
C13 - C14 - C15 = 120.5
C14 - C15 - C16 = 119.9
C15 - C16 - C17 = 120.2
C16 - C17 - C18 = 120.0
C13 - C18 - C17 = 120.2

Abbildung 2:

Bindungszustände und Bindungswinkel in (E)-2-Amino-5-chlorbenzophenon-amidinohydrazonacetat

In der Kristallstruktur des mit Ethanol und Wasser kristallisierten (Z)-2-Amino-5-chlorbenzophenonamidinohydrazonacetats sind die Wirkstoff-, Acetat-, Ethanol- und Wassermoleküle durch ein zweidimensionales Netzwerk von Wasserstoffbrücken (9 symmetrieunabhängige H-Brücken) miteinander verknüpft. Für die Ethanolmoleküle wurde eine Lagefehlordnung beobachtet, die für die Zersetzung der Kristalle an der Luft unter Verlust von Ethanol und Wasser verantwortlich zu machen ist. Das dabei entstehende Pulver ist identisch mit der kristallinen Modifikation des (Z)-2-Amino-5-chlorbenzophenonamidinohydrazonacetats.

Das (E)-2-Amino-5-chlorbenzophenonamidinohydrazonacetat weist außer einer intramolekularen $N_6$-H...N8-Brücke vier symmetrieunabhängige Wasserstoffbrücken auf, die die Wirkstoff- und Acetatmoleküle zu Ketten verknüpfen.

Da sich die auf diese Weise eindeutig als Z- bzw. E-Isomere charakterisierten Verbindungen auch hinsichtlich ihres UV-spektroskopischen und dünnschichtchromatografischen Verhaltens deutlich voneinander unterscheiden, sich aber jeweils alle Z- bzw. alle E-Isomere der substituierten 2-Aminobenzophenonamidinohydrazone in ihren Eigenschaften weitgehend ähneln, gestatten die UV-spektroskopischen und dünnschichtchromatrografischen Angaben eine relativ einfache sterische Zuordnung der jeweils vorliegenden Verbindung (s.Ausführungsbeispiele).

TABELLE 1

Atomkoordinaten (.$10^4$) der Nichtwasserstoffatome in (Z)-2-Amino-5-chlorbenzophenonamidinohydrazonacetat.$C_2H_5OH.H_2O$ und (E)-2-Amino-5-chlorbenzophenonamidinohydrazonacetat (Standardabweichung in Klammern)

| Atom | (Z)-$C_{16}H_{15}ClN_5O_2.C_2H_5OH.H_2O$ | | | (E)-$C_{16}H_{15}ClN_5O_2$ | | |
|---|---|---|---|---|---|---|
| | x/a | y/b | z/c | x/a | y/b | z/c |
| C13 | 4868(2) | 874(2) | 3007(1) | 6903(1) | 4418(1) | 6433(1) |
| N6 | 8456(5) | 493(6) | 1122(2) | 5443(2) | 7154(4) | 8891(1) |
| N8 | 7920(4) | 3948(4) | 1148(2) | 4620(1) | 10216(4) | 7870(1) |
| N9 | 8099(4) | 4179(4) | 1651(2) | 3949(1) | 11692(4) | 7596(1) |
| N11 | 8813(5) | 6215(5) | 1222(2) | 4274(1) | 12981(4) | 8852(1) |
| N12 | 9589(5) | 5658(5) | 2157(2) | 3080(2) | 14235(4) | 7807(1) |
| C1 | 6684(5) | 1697(5) | 1578(2) | 5483(2) | 7545(4) | 7559(1) |
| C2 | 5862(5) | 1783(6) | 2024(2) | 5855(2) | 6859(4) | 6994(1) |
| C3 | 5929(6) | 766(7) | 2450(2) | 6446(2) | 5227(4) | 7147(2) |
| C4 | 6816(6) | -350(6) | 2437(2) | 6667(2) | 4210(1) | 7848(2) |
| C5 | 7635(6) | -435(6) | 1997(3) | 6312(2) | 4861(5) | 8410(2) |
| C6 | 7608(5) | 583(6) | 1555(2) | 5732(2) | 6556(4) | 8249(1) |
| C7 | 6612(5) | 2797(5) | 1114(2) | 4824(2) | 9237(4) | 7336(1) |
| C10 | 8845(5) | 5378(6) | 1673(2) | 3771(2) | 12989(4) | 8089(1) |
| C13 | 5748(6) | 2547(6) | 577(2) | 4398(2) | 9732(4) | 6479(1) |
| C14 | 5941(9) | 3299(8) | 79(3) | 3703(2) | 8535(5) | 5979(2) |
| C15 | 5105(10) | 3087(9) | -421(39) | 3314(2) | 8992(6) | 5187(2) |
| C16 | 4102(8) | 2104(8) | -443(3) | 3623(2) | 10622(6) | 4892(2) |
| C17 | 3903(7) | 1354(8) | 44(3) | 4307(2) | 11837(6) | 5383(2) |

| | | | | | | |
|---|---|---|---|---|---|---|
| C18 | 4738(6) | 1553(7) | 555(3) | 4694(2) | 11391(5) | 6178(2) |
| O1A | 8848(4) | 1827(4) | 7801(2) | 2200(1) | 12366(3) | 6315(1) |
| O2A | 9208(4) | 1738(5) | 8750(2) | 1091(1) | 11230(3) | 5248(1) |
| C1A | 8502(5) | 1511(6) | 8292(2) | 1708(2) | 12458(4) | 5602(1) |
| C2A | 7184(7) | 763(9) | 8329(3) | 1876(2) | 14212(5) | 5144(2) |
| O1E | 660 | 3922 | 149 | | | |
| C2E | 1689 | 4151 | 629 | | | |
| C3E | 1386 | 3042 | 1052 | | | |
| O1W | 9304 | 3720 | 4914 | | | |

TABELLE 2

| Pulverdiffraktionsdiagramm von (Z)-2-Amino-5-chlorbenzophenonamidinohydrazonacetat (krist.) (Cuk-Strahlung) | | | | | |
|---|---|---|---|---|---|
| d(A) | $I/I_o$ | d(A) | $I/I_o$ | d(A) | $I/I_o$ |
| 8,401 | 29 | 5,102 | 3 | 3,814 | 46 |
| 8,072 | 46 | 4,896 | 16 | 3,746 | 30 |
| 7,602 | 4 | 4,622 | 70 | 3,673 | 45 |
| 7,047 | 15 | 4,320 | 51 | 3,583 | 4 |
| 6,237 | 14 | 4,278 | 30 | 3,485 | 26 |
| 6,034 | 17 | 4,101 | 45 | 3,381 | 33 |
| 5,879 | 5 | 4,020 | 26 | 3,274 | 30 |
| 5,784 | 6 | 3,941 | 7 | 3,265 | 26 |
| 5,448 | 100 | | | | |

Ausführungsbeispiele

Beispiel 1

2-Aminobenzophenonhydrazon

0,1 Mol 2-Aminobenzophenon werden unter Erwärmen in 200 ml Ethanol gelöst und die Lösung nach Zusatz von 58 g Hydrazinhydratlösung (85%) und 2 g Toluen-4-sulfonsäure auf dem Wasserbad 50 h unter Rückfluß erhitzt. Beim Abkühlen der Reaktionslösung kristallisiert das Hydrazon aus. Es wird abgesaugt, mit verd. Ethanol gewaschen und nach dem Trock nen aus Ethanol umkristallisiert. Durch Einengen der filtrierten Reaktionslösung kann eine weitere Fraktion erhalten werden.
Ausbeute: 55% Farblose Kristalle Schmb.: 134-143° C (Ethanol).
$C_{13}H_{13}N_3$ (211,3) Ber.: N 19,88 Gef.: N 20,09
Analog können erhalten werden:
2-Amino-5-chlorbenzophenonhydrazon
Ausbeute: 70% Farblose Kristalle Schmb.: 133-134° C (Ethanol).
$C_{13}H_{12}ClN_3$ (245,7) Ber.: N 17,10 Gef.: N17,35
2-Amino-5-nitrobenzophenonhydrazon
Ausbeute: 45% Gelbe Kristalle Schmb.: 179-182° C (Ethanol)
$C_{13}H_{12}N_4O_2$ (256,3) Ber.: N 16,40 Gef.: N 16,25

Beispiel 2

(E)-/(Z)-2-Aminobenzophenonamidinohydrazonnitrat

Jeweils 0,1 Mol des 2-Aminobenzophenonhydrazons und des 1-Amidino-3,5-dimethylpyrazolnitrats (J.chem.Soc. (London) 74, 3456 (1952); 75, 4053 (1953) ) werden in 200 ml Ethanol unter Erwärmen gelöst und die Lösung anschließend unter Rückfluß erhitzt. Nach 30h wird die Reaktionslösung i.Vak. zur Trockne eingeengt, der Rückstand nach Verreiben mit Ether in Ethanol gelöst und die Lösung bis zur starken Trübung mit Ether versetzt. Die nach einigem Stehen abgeschiedenen Kristalle werden abgesaugt und mit wenig Ether gewaschen.
Ausbeute: 40% Gelbliche Kristalle Schmb.: 189-196° C (Ethanol/Ether).
$C_{14}H_{16}N_6O_3$ (316,3) Ber.: N 25,57 Gef.: N 26,83

Analog kann erhalten werden:
(E)-/(Z)-2-Amino-5-chlorbenzophenonamidinohydrazonnitrat
Ausbeute: 47% Gelbliche Kristalle Schmb.: ab 232° C u. Zers.($H_2O$)
$C_{14}H_{15}ClN_6O_3$ (350,8) Ber.: N 23,95 Gef.: N 23,64

Beispiel 3

(E)-/(Z)-2-Amino-5-chlorbenzophenon-N-phenylamidinohydrazonnitrat

0,1 Mol 2-Amino-5-chlorbenzophenonhydrazon und 0,12 Mol Phenylcyanamid werden in 100 ml abs. Dioxan gelöst und die Lösung 20 h auf dem siedenden Wasserbad erhitzt. Danach wird die Reaktionslösung i. Vak. bis fast zur Trockne eingeengt und der Rückstand in 50 ml Ethanol aufgenommen. Nach Filtration wird die Lösung bis zur deutlich sauren Reaktion mit konz.Salpetersäure und danach mit Ether bis zur starken Trübung versetzt. Die nach einigem Stehen abgeschiedenen Kristalle werden abgesaugt. Zur Reinigung wird aus Ethanol/Ether umgefällt.
Ausbeute: 40% Gelbliche Kristalle Schmb.: 180-183° C (Ethanol/Ether).
$C_{20}H_{15}N_6O_3$ (426,9) Ber.: N 19,68 Gef.: N 19,27

Analog kann erhalten werden:
(E)-/(Z)-2-Amino-5-nitro-N-phenylamidinohydrazonnitrat
Ausbeute: 28% Gelbe Kristalle Schmb.: 210-212° C (Ethanol/Ether).
$C_{20}H_{19}N_7O_5$ (437,4) Ber.: N 22,42 Gef.: N 22,63

Beispiel 4

2-Amino-5-chlorbenzophenonthiosemicarbazon

0,1 Mol 2-Amino-5-chlorbenzophenon, 4,0 g Toluen-4-sulfonsäure und 0,11 Mol Thiosemicarbazid werden in einer Mischung von 250 ml Methanol und 50 ml $H_2O$ gelöst und die Lösung 16h unter Rückfluß erhitzt. Die nach dem Abkühlen des Ansatzes abgeschiedenen Kristalle werden abgesaugt und aus Propan-1-ol umkristallisiert.
Ausbeute: 75-80% Weiße Kristalle Schmb. 206-208° C (Propan-1-ol)

| | | | |
|---|---|---|---|
| $C_{14}H_{13}ClN_4S$ (304,8) | Ber.: | N 18,38 | S 10,52 |
| | Gef.: | N 18,23 | S 10.81 |

Beispiel 5

2-Amino-5-chlorbenzophenon-(4-phenyl)-thiosemicarbazon

0,1 Mol 2-Amino-5-chlorbenzophenon, 4.0 g Toluen-4-sulfonsäure und 0,11 Mol 4-Phenylthiosemicarbazid werden in 250 ml Methanol gelöst und die Lösung 16h unter Rückfluß erhitzt. Ausfallende Nebenprodukte werden nach 8-stündigem Erhitzen abfiltriert und die filtrierte Lösung weitererhitzt.

Die nach dem Abkühlen des Ansatzes abgeschiedenen Kristalle werden abgesaugt und aus Propan-1-ol

umkristallisiert.
Ausbeute: 65-70 % Weiße Kristalle Schmb.: 159-161 °C (Propan-1-ol)

| | | | | |
|---|---|---|---|---|
| $C_{20}H_{17}ClN_4S$ (380,9) | Ber.: | C 63,07 | H 4,50 | N 14,71 |
| | Gef.: | C 63,25 | H 4,74 | N 17,59 |

Beispiel 6

(E)-/(Z)-2-Amino-5-chlorbenzophenonamidinohydrazonnitrat

0,1 Mol 2-Amino-5-chlorbenzophenonthiosemicarbazon werden unter gelindem Erwärmen in 200 ml abs. Dioxan gelöst. Nach Zusatz von 0,12 Mol Iodmethan wird die Lösung 24h bei Raumtemperatur stehengelassen. Danach wird sie i.Vak. eingeengt und der Rückstand in 100 ml Ethanol aufgenommen. Nach Einleiten von Ammoniak bei Raumtemperatur wird die Lösung ggf. von ausgeschiedenen Kristallen dekantiert und danach unter ständigem Einleiten von Ammoniak 8h auf 70 bis 75 °C erhitzt. Anschließend wird der Ansatz auf ein Drittel seines Volumens eingeengt und mit konz. Salpetersäure bis zur deutlich sauren Reaktion versetzt. Das beim Abkühlen der Lösung kristallisierende Produkt wird abgesaugt und aus Ethanol/Ether umgefällt. Ausbeute : 45%

Analog kann erhalten werden:
(E)-/(Z)-2-Amino-5-chlorbenzophenon-N-phenylamidinohydrazonnitrat
Ausbeute: 40%

Beispiel 7

2-Amino-5-chlorbenzophenon-(methylthio)-thiocarbonylhydrazon

0,1 Mol 2-Amino-5-chlorbenzophenon, 0,1 Mol Hydrazindithiocarbonsäuremethylester (S.M. Losanitsch, J.chem. Soc. (London) 119, 764) und 4,0 g Toluen-4-sulfonsäure werden in 300 ml Methanol gelöst und die Lösung 8h unter Rückfluß erhitzt. Nach dem Abkühlen wird das Produkt ggf. durch Anreiben zur Kristallisation gebracht, abgesaugt und umkristallisiert. Ausbeute: 60%
Gelbe Nadeln Schmb.: 161-162 °C (Methanol)

| | | | | |
|---|---|---|---|---|
| $C_{15}H_{14}ClN_3S_2$ (335,9) | Ber.: | C 53,62 | H 4,20 | N 12,51 |
| | Gef.: | C 53,87 | H 4,03 | N 12,49 |

Beispiel 8

(E)-(Z)-2-Amino-5-chlorbenzophenonamidinohydrazonnitrat

0,1 Mol 2-Amino-5-chlorbenzophenon-(methylthio)-thiocarbonylhydrazon werden unter gelindem Erwärmen in 350 ml abs. Dioxan gelöst. Nach Zusatz von 0,25 Mol Iodmethan wird der Ansatz 48h bei Raumtemperatur stehengelassen. Danach wird die Lösung i.Vak. eingeengt und der Rückstand in 100 ml Ethanol aufgenommen. Nach Einleiten von Ammoniak bei Raumtemperatur wird die Lösung ggf. von ausgeschiedenen Kristallen dekantiert und danach unter ständigem Einleiten von Ammoniak 8h auf 70 bis 75 °C erhitzt. Anschließend wird der Ansatz auf ein Drittel seines Volumens eingeengt und mit konz. Salpetersäure bis zur deutlich sauren Reaktion versetzt. Das beim Abkühlen der Lösung auskristallisierende Produkt wird abgesaugt und aus Ethanol/Ether umgefällt.
Ausbeute: 53%

Beispiel 9

(Z)/(E)-2-Amino-5-chlorbenzophenonamidinohydrazondihydrochlorid

1 Mol 2-Amino-5-chlorbenzophenon wird unter Erwärmen in 190 ml Ethanol gelöst und unter Rühren mit einer Lösung von 1,2 Mol Aminoguanidinhydrochlorid in 100 ml Wasser und danach mit 236 ml konz. Salzsäure versetzt. Der Ansatz wird 8 h auf dem Wasserbad zum Sieden erhitzt.
Danach läßt man den Reaktionsansatz völlig abkühlen. Anschließend wird das Produkt abgesaugt, mit wenig Methanol und Ether gewaschen und getrocknet.
Ausbeute: 90% Farblose Kristalle Schmb.: ab 182 °C (Verfärbung ab 160 °C)
$C_{14}H_{16}Cl_3N_5$ (360,7) Ber.: N 19,41 Gef.: N 19,09

Analog können weitere Salze substituierter (Z)-/(E)-2-Aminobenzophenonamidinohydrazone erhalten werden (vgl. Tabelle 3).
Einige der Reaktionsprodukte kristallisieren aus den Ansätzen aus. In anderen Fällen macht sich ein Einengen der Lösung i.Vak. auf etwa ein Drittel ihres Volumens und ggf. eine Zugabe von Ether zum Konzentrat erforderlich, um die Produkte abzuscheiden. Diese werden abgesaugt, mit wenig Ethanol und anschließend mit Aceton bzw. Ether gewaschen und getrocknet. Eine Reinigung kann durch Umkristallisation bzw. Umfälung erfolgen.

# TABELLE 3

weitere (E)-/(Z)-2-Aminobenzophenonamidinohydrazonhydrochloride

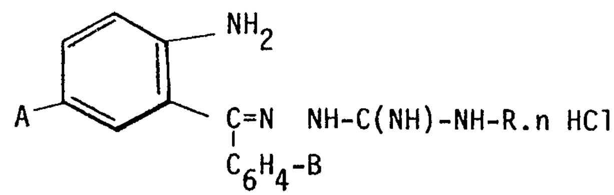

| Nr. | A | B | R | n | Ausb. (%) | Summenformel (Molmasse) | N ber. N gef. | Schmb. °C (Umkrist.) | Acon.-arrh. i.v. $-ED_{20}$ (mol/kg) |
|---|---|---|---|---|---|---|---|---|---|
| 1 | H | H | H | 2 | 41 | $C_{14}H_{17}Cl_2N_5$ (326,2) | 21,47 21,62 | 207-210 (Ethanol) | |
| 2 | H | H | $C_6H_5$ | 2 | 45 | $C_{20}H_{21}Cl_2N_5$ (402,3) | 17,41 17,48 | 165-176 (Ethanol/Ether) | $2,7 \cdot 10^{-6}$ |
| 3 | $NH_2$ | H | H | 3 | 42 | $C_{14}H_{19}Cl_3N_6$ (377,7) | 22,25 22,23 | 199-212 (Ethanol) | |
| 4 | Br | H | H | 2 | 37 | $C_{14}H_{16}BrCl_2N_5$ (405,1) | 17,29 17,40 | 292-295 (Ethanol) | $3,2 \cdot 10^{-6}$ |
| 5 | Cl | 4-OH | H | 2 | 85 | $C_{14}H_{16}Cl_3N_5O$ (376,7) | 18,59 18,33 | 185-194 (Ethanol/Ether) | |

TABELLE 3

Fortsetzung

| Nr. | A | B | R | n | Ausb. (%) | Summenformel (Molmasse) | N ber. N gef. | Schmb. °C (Umkrist.) | Acon.-arrh. i.v. -ED (mol/kg) |
|---|---|---|---|---|---|---|---|---|---|
| 6 | H | 3-Cl | H | 1 | 90 | $C_{14}H_{15}Cl_2N_5$ (324,2) | 21,60 21,67 | 170-172 (Ethanol/Ether) | $4,3 \cdot 10^{-6}$ |
| 7 | H | 4-Cl | H | 2 | 61 | $C_{14}H_{16}Cl_3N_5$ (360,7) | 19,42 19,45 | 248-250 Tr. ab 180 (Ethanol) | $6,2 \cdot 10^{-6}$ |
| 8 | H | 4-$CH_3$ | H | 2 | 58 | $C_{15}H_{19}Cl_2N_5$ (340,3) | 20,58 20,74 | ab 169 (Ethanol) | $3,2 \cdot 10^{-6}$ |
| 9 | $CH_3$ | H | H | 2 | 40 | $C_{15}H_{19}Cl_2N_5$ (340,3) | 20,58 21,03 | 265-272 Tr. ab 169 (Propan-2-ol) | |
| 10 | $NO_2$ | 2-Cl | H | 1 | 52 | $C_{14}H_{14}Cl_2N_6O_2$ (369,2) | 22,76 22,73 | 305-314 (Ethanol/Ether) | |

Beispiel 10

(Z)-1-Amino-5-chlorbenzophenonamidinohydrazondihydrochlorid

1 Mol 2-Amino-5-chlorbenzophenon wird unter Erwärmen in 190 ml Ethanol gelöst und unter Rühren mit einer Lösung von 1,2 Mol Aminoguanidinhydrochlorid in 100 ml Wasser und danach mit 236 ml konz.Salzsäure versetzt. Der Ansatz wird 8 h auf dem Wasserbad zum Sieden erhitzt. Danach wird das aus dem heißen Reaktionssatz auskristallisierte Produkt abgesaugt, mit wenig Methanol und Ether gewaschen und anschliessend getrocknet.
Ausbeute: 45% Farblose Kristalle Schmb.: 288-296 °C und Zers. (Ethanol) (Verfärbung ab 160 °C)
$C_{14}H_{16}Cl_3N_5$ (360,7) Ber.: N 19,41 Gef.: N 19,21
UV [$\lambda_{max}$(nm) (lg$\epsilon$) (Methanol)]; 251 (4,26); 283 (4,31)
Analog kann erhalten werden:
(Z)-2-Amino-2′,5-dichlorbenzophenonamidinohydrazondihydrochlorid
Ausbeute: 30% Schwach gelbliche Nadeln Schmb.: 251-266 °C (Ethanol)
$C_{14}H_{15}Cl_4N_5$ (395,1) Ber. N 17,72 Gef.: N 17,52
UV [$\lambda_{max}$(nm) (lg$\epsilon$) (Methanol)]; 252 (4,38); 279 (4,18)

Beispiel 11

(Z)-2-Amino-5-chlorbenzophenonamidinohydrazondihydrochlorid.$C_2H_5OH$

Das nach Beispiel 10 erhaltene Rohprodukt kann aus Ethanol umkristallisiert werden. Die Verbindung kristallisiert dann mit 1 Mol Ethanol aus.
Farblose Kristalle Schmb.: 280-282 °C u. Zers. (Ethanol)
$C_{14}H_{16}Cl_3N_5$ . $C_2H_5OH$ (406,7) Ber.: N 17,22 Gef.: N 17,52

Beispiel 12

(E)-2-Amino-5-chlorbenzophenonamidinohydrazondihydrochlorid

Das Filtrat des heißen Reaktionssatzes nach Beispiel 10 wird stehengelassen. Nach Abkühlen auf Raumtemperatur wird das auskristallisierte Produkt abgesaugt, mit wenig Methanol und Ether gewaschen und umkristallisiert. Ausbeute: 47% Farblose Kristalle Schmb.: 194-197 °C u. Zers. (Ethanol/konz. Salzsäure) (Verfärbung ab 160 °C, Tröpfchen ab 180 °C)
$C_{14}H_{16}Cl_3N_5$ (360,7) Ber.: N 19,41 Gef.: 19,10
UV [$\lambda_{max}$(nm) (lg$\epsilon$) (Methanol)]; 231 (4,37); 253 (4,31); 280 (4,06) (sh); 372 (3,72)
Analog können erhalten werden:
(E)-2-Amino-2′,5-dichlorbenzophenonamidinohydrazondihydrochlorid
Ausbeute: 35% Gelbliche Nadeln Schmb.: 179-196 °C (Ethanol)
$C_{14}H_{15}Cl_4N_5$ (395,1) Ber.: N 17,72 Gef.: N 17,69
UV [$\lambda_{max}$(nm) (lg$\epsilon$) (Methanol)]; 253 (4,40); 279 (4,13); 370 (3,55)
(E)-2-Amino-5-nitrobenzophenonamidinohydrazonhydrochlorid
Ausbeute: 67% Gelbe Kristalle Schmb.: 254-257 °C, Tröpfchen ab 191 °C (Ethanol)
$C_{14}H_{15}ClN_6O_2$ (334,8) Ber.: N 25,10 Gef.: N 25,31
UV [$\lambda_{max}$(nm) (lg$\epsilon$) (Methanol)]; 285 (4,23); 362 (4,08)

Beispiel 13

(Z)-2-Amino-5-chlorbenzophenonamidinohydrazon

0,5 Mol (Z)-2-Amino-5-chlorbenzophenonamidinohydrazondihydrochlorid werden in 3400 ml Wasser suspendiert und unter ständigem Rühren mit 500 ml NaOH (3 Mol.$1^{-1}$) versetzt. Das Rühren wird 1h bei Raumtemperatur fortgesetzt. Danach wird abgesaugt, mit Wasser gründlich gewaschen, unter Anpressen abgesaugt und getrocknet.
Ausbeute: 85% Amorphes farbloses Pulver Schmb.: 176-184 °C

$C_{14}H_{14}ClN_5$ (287,8) Ber.: N 24,34 Gef.: N 24,34

Analog können erhalten werden:

(Z)-2-Amino-5-chlorbenzophenon-N-phenylamidinohydrazon

Ausbeute: 70% Amorphes farbloses Pulver Schmb.: 189-192° C

| | | | |
|---|---|---|---|
| $C_{20}H_{15}ClN_5$ (363,8) | Ber.:<br>Gef.: | C 66,02<br>C 65,72 | N 19,25<br>N 19,20 |

UV [$\lambda_{max}$(nm) (lg$\epsilon$) (Methanol)]; 253 (4,26) (sh); 299 (4,22); 339 ( 4,05)

(E)-2-Amino-5-chlorbenzophenonamidinohydrazon

0,5 Mol des Hydrochlorids werden in 1500 ml Wasser suspendiert und, wie vorstehend beschrieben, weiterbehandelt.

Ausbeute: 85% Amorphes farbloses Pulver Schmb.: 176-184° C

$C_{14}H_{14}ClN_5$ (287,8) Ber.: N 24,34 Gef.: N 24,34

Analog können erhalten werden:

(Z)-2-Amino-5-chlorbenzophenon-N-phenylamidinohydrazon

Ausbeute: 70% Amorphes farbloses Pulver Schmb.: 189-192° C

| | | | |
|---|---|---|---|
| $C_{20}H_{15}ClN_5$ (363,8) | Ber.:<br>Gef.: | C 66,02<br>C 65,72 | N 19,25<br>N 19,20 |

UV [$\lambda_{max}$(nm) (lg$\epsilon$) (Methanol)]; 253 (4,26) (sh); 299 (4,22); 339 (4,05)

(E)-2-Amino-5-chlorbenzophenonamidinohydrazon

0,5 Mol des Hydrochlorids werden in 1500 ml Wasser suspendiert und, wie vorstehend beschrieben, weiterbehandelt.

Ausbeute: 75% Amorphes gelbes Pulver Schmb.: 174-194° C

$C_{14}H_{14}ClN_5$ (287,8) Ber.: N 24,34 Gef.: N 24,53

(E)-2-Amino-5-chlorbenzophenon-N-phenylamidinohydrazon

Ausbeute: 85% Amorphes hellgelbes Pulver Schmb. 158-164° C

| | | | | |
|---|---|---|---|---|
| $C_{20}H_{15}ClN_5$ (363,8) | Ber.:<br>Gef.: | C 66,02<br>C 66,36 | H 4,99<br>H 4,96 | N 19,25<br>N 19,71 |

UV [$\lambda_{max}$(nm) (lg$\epsilon$) (Methanol)]; 236 (4,41) (Sh); 292 (4,18) (sh); 371 (4,05)

Analog können auch aus den in den Beispielen 2, 3, 6 und 8 beschriebenen Nitraten sowie aus den in Tabelle 1 aufgeführten Dihydrochloriden die entsprechenden Basen freigesetzt werden.

Beispiel 14

(Z)-2-Acetamino-5-chlorbenzophenonamidinohydrazonhydrochlorid. $H_2O$

1,11 Mol des nach Beispiel 9 erhaltenen (E)-/(Z)-2-Amino-5-chlorbenzophenonamidinohydrazond ihydrochlorid-Gemisches werden in 600 ml Toluen suspendiert und die Suspension bei einer Innentemperatur von 20 - 30° C mit 2,56 Mol Acetanhydrid innerhalb 25 min versetzt. Danach wird der Ansatz innerhalb von 30 min zum Sieden gebracht und 1h unter Rückfluß erhitzt. Anschließend werden i.Vak. ca. 500 ml Flüssigkeit abdestilliert. In das noch rührfähige Gemisch werden nun 400 ml Wasser mit einer Temperatur von 50 - 60° C schnell eingetragen. Nach Abkühlen auf unter 20° C isoliert man ein weißes Produkt, das bis zur Gewichtskonstanz bei 100° C getrocknet wird. Ausbeute: 80%

Weiße Kristalle Schmb.: 178-182° C (Ethanol)

| $C_{16}H_{17}Cl_2N_5O \cdot H_2O$ (384,3) | | | |
|---|---|---|---|
| Ber.: | N 18,23 | Cl 18,45 | Cl (ionisch) 9,23 |
| Gef.: | N 18,07 | Cl 18,47 | Cl (ionisch) 9,24 |

$^1$H-NMR (DMF, $\delta$ in ppm); 1,91 s ($CH_3$); 3,68 t ($H_2O$)
UV [$\lambda_{max}$(nm) (lg$\epsilon$) (Methanol)]; 221 (4,38); 282 (4,25)

Beispiel 15

(Z)-2-Amino-5-chlorbenzophenonamidinohydrazondihydrochlorid.$C_2H_5OH$

0,34 Mol des nach Beispiel 14 erhaltenen (Z)-2-Acetamino-5-chlorbenzophenonamidinohydrazonhydrochloridhydrats werden unter Erhitzen in 900 ml 96%igem Ethanol gelöst. Die zum Sieden unter Rückfluß erhitzte Lösung wird mit 45 ml konz. Salzsäure versetzt, nach 1h werden erneut 45 ml und nach 5h nochmals 70 ml konz. Salzsäure zugesetzt. Danach wird noch 2h weitererhitzt.
Anschließend wird der Ansatz abgekühlt, der entstandene Niederschlag wird abgesaugt, zweimal mit je 50 ml Aceton gewaschen und danach bei 60 °C im Trockenschrank getrocknet. Ausbeute: 63%
Farblose Kristalle Schmb.: 280-282 °C u. Zers. (Ethanol)

| $C_{14}H_{16}Cl_3N_5 \cdot C_2H_5OH$ (406,7) | | |
|---|---|---|
| Ber.: | N 17,22 | Cl 26,14 |
| Gef.: | N 17,50 | Cl 26,70 |

$^1$H-NMR (DMF, $\delta$ in ppm); 1,17 s ($CH_3$); 3,6 g ($OCH_2$)
UV [$\lambda_{max}$(nm) (lg$\epsilon$) (Methanol)]; 249(4,29); 282(4,33)

Beispiel 16

(Z)-2-Amino-5-chlorbenzophenonamidinohydrazondihydrochlorid

0,28 Mol (E)- (Beispiel 12) bzw. (E)-/(Z)-2-Amino-5-chlorbenzophenonamidinohydrazondihydrochlorid (Beispiel 9) werden in 100 ml Toluen eingerührt und die Suspension bei einer Innentemperatur von 20-25 °C mit 0,64 Mol Acetanhydrid versetzt. Innerhalb von 15 min wird zum Rückfluß, der bei 85 °C beginnt, aufgeheizt und weitere 30 min unter Rückfluß erhitzt. Die Innentemperatur steigt während dieser Zeit bis auf 103 °C an.
Anschließend destilliert man i.Vak. das gesamte Lösungsmittel ab und löst den sirupösen Rückstand in 500 ml heißem 96%igen Ethanol. Nach erneutem Erhitzen zum Rückfluß werden 20 ml konz. Salzsäure und danach im Abstand von jeweil 1h weitere 60 ml konz. Salzsäure in 15 ml-Portionen eingetragen. Die gesamte Reaktionszeit für die Hydrolyse beträgt 7h. Die Aufarbeitung erfolgt analog Beispiel 15. Ausbeute: 71%

Beispiel 17

(Z)-2-Amino-5-chlorbenzophenonamidinohydrazondihydrochlorid

0,28 Mol (E)- (Beispiel 12) bzw. (E)-/(Z)-2-Amino-5-chlorbenzophenonamidinohydrazondihydrochlorid (Beispiel 9) werden in 150 ml Toluen eingerührt und die Suspension auf eine Innentemperatur von 90 °C erhitzt. Unter ständigem Rühren werden anschließend 0,64 Mol Acetanhydrid innerhalb von 15 min zugetropft, wobei es nach einem kurzzeitigen Temperaturanstieg auf 98 °C zum Temperaturabfall kommt. Das Gemisch wird min unter Rückfluß erhitzt und danach analog Beispiel 16 weiterbehandelt. Ausbeute: 70%

Beispiel 18

(Z)-2-Amino-5-chlorbenzophenonamidinohydrazondihydrochlorid

0,28 Mol (E)- (Beispiel 12) bzw. (E)/(Z-2-Amino-5-chlorbenzophenonamidinohydrazondihydrochlorid (Beispiel 9) werden in 150 ml Ethylacetat suspendiert und die Suspension mit 0,64 Mol Acetanhydrid versetzt. Nach langsamem Erwärmen auf Rückflußtemperatur wird der Ansatz 30 min unter Rückfluß erhitzt und danach analog Beispiel 16 weiterbehandelt. Ausbeute 60%

Beispiel 19

(Z)-2-Amino-5-chlorbenzophenonamidinohydrazondihydrochlorid

0,28 Mol (E)- (Beispiel 12) bzw. (E)-/(Z)-2-Amino-5-chlorbenzophenonamidinohydrazondihydrochlorid (Beispiel 9) werden in 150 ml Toluen suspendiert und die Suspension mit 60 ml Acetanhydrid und 20 ml Acetylchlorid versetzt. Der Ansatz wird innerhalb von 15 min zum Sieden gebracht und 30 min unter Rückfluß erhitzt. Danach wird das Lösungsmittel i.Vak. abdestilliert. Den Rückstand löst man in 500 ml siedendem Ethanol. Die siedende Lösung versetzt man zunächst mit 30 ml, nach 1h mit weiteren 30 ml und nach 4h nochmals mit 20 ml konz.Salzsäure. Danach wird der Ansatz noch 1h unter Rückfluß erhitzt, abgekühlt und anschließend der entstandene Niederschlag abgesaugt, mit Aceton gewaschen und bei 60 °C getrocknet. Ausbeute 43%

Beispiel 20

(Z)-2-Amino-5-chlorbenzophenonamidinohydrazondihydrochlorid

0,028 Mol (E)- (Beispiel 12) bzw. (E)-/(Z)-2-Amino-5-chlorbenzophenonamidinohydrazondihydrochlorid (Beispiel (9) werden in 40 ml Dimethylformamid und 10 ml konz. Salzsäure gelöst und auf 80 °C erhitzt. Der Ansatz wird 14h bei dieser Temperatur gerührt. Danach wird die Lösung i. Vak. auf ein Drittel ihres Volumens eingeengt und mit 200 ml Wasser versetzt. Beim Abkühlen unter ständigem Rühren scheidet sich ein Niederschlag ab, der abgesaugt, mit Aceton gewaschen und getrocknet wird. Ausbeute 60%

Beispiel 21

(Z) -2-Amino-5-chlorbenzophenonamidinohydrazonacetat

0,5 Mol (Z)-2-Amino-5-chlorbenzophenonamidinohydrazon werden in ca. 500 ml Aceton unter gelindem Erwärmen gelöst. Die ggf. filtrierte Lösung wird mit 0,5 Mol Essigsäure versetzt und das Acetat durch Anreiben zur Kristallisation gebracht. Es wird abgesaugt, mit wenig Aceton und Ether gewaschen und getrocknet.
Ausbeute: 90% Farblose Kristalle Schmb.: 164-170 °C (Tröpfchen ab 130 °C) (Aceton)
$C_{16}H_{15}ClN_5O_2$ (347,8) Ber.: N 20,13 Gef.: N 20,33
UV [$\lambda_{max}$(nm) (lg$\epsilon$) (Methanol)]; 251 (4,28; 283 (4,32)
Analog kann erhalten werden:

(Z)-2-Amino-5-chlorbenzophenon-N-phenylamidinohydrazonacetat
Ausbeute: 65% Farblose Nadeln Schmb.: 131-136 °C (Ethanol/Eisessig)

| | | | | |
|---|---|---|---|---|
| $C_{22}H_{22}ClN_5O_2$ (423,9) | Ber.: | C 62,34 | H 5,23 | N 16,52 |
| | Gef.: | C 61,85 | H 5,15 | N 16,42 |

UV [$\lambda_{max}$(nm) (lg$\epsilon$) (Methanol)]; 226 (4,34; 250 (4,26) (sh); 291 (4,32)
Analog können weitere (Z)-2-Amino-5-chlorbenzophenonamidinohydrazonsalze erhalten werden (s.Tabelle 4).
Sie kristallisieren entweder beim Abkühlen der Lösung oder nach Zusatz von Ether und Anreiben aus. Tartrate und Citrate werden häufig zunächst in sirupöser Form abgeschieden, werden aber bei mehrmaligem Verreiben mit Ether fest. Die Salze werden abgesaugt und umgefällt bzw. umkristallisiert.

# TABELLE 4

weitere (Z)-2-Amino-5-chlorbenzophenonamidinohydrazonsalze

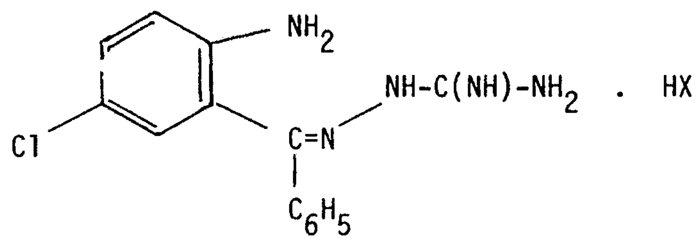

| Nr. | HX | Ausb. (5) (bez. a. Base) | Summenformel (Molmasse) | Nr. ber. N gef. | Schm. °C (Umkrist.) |
|---|---|---|---|---|---|
| 1 | $C_2H_5COOH$ | 22 | $C_{17}H_{20}ClN_5O_2$ (361,8) | 19,36 19,43 | 85 - 97 (Aceton) |
| 2 | $C_3H_7COOH$ | 25 | $C_{18}H_{22}ClN_5O_2$ (375,9) | 18,63 18,21 | 110 - 112 (Aceton) |
| 3 | $C_4H_9COOH$ | 10 | $C_{19}H_{24}ClN_5O_2$ (389,9) | 17,96 17,76 | 100 - 105 (Aceton) |
| 4 | $HOOC-CH_2-CH_2-COOH$ | 60 | $C_{18}H_{20}ClN_5O_4$ (405,8) | 17,26 17,34 | 197 - 199 (Methanol) |
| 5 | HOOC-CH-=CH-COOH (Z) | 55 | $C_{18}H_{18}ClN_5O_4$ (403,8) | 17,35 17,13 | 185 - 189 (Methanol) |

TABELLE 4

Fortsetzung

| Nr. | HX | Ausb. (5) (bez. a. Base) | Summenformel (Molmasse) | Nr. ber. N gef. | Schm. °C (Umkrist.) |
|---|---|---|---|---|---|
| 6 | HOOC-CHOH-CHOH-COOH | 35 | $C_{18}H_{20}ClN_5O_6$<br>(437,8) | 16.00<br>15,83 | ab 124 u. Z.<br>(Methanol/Ether) |
| 7 | OH<br>HOOC-$CH_2$-C-$CH_2$-COOH<br>COOH | 30 | $C_{20}H_{22}ClN_5O_7$<br>(479,9) | 14,59<br>14,56 | ab 139 u.Z.<br>(Methanol) |
| 8 | $CH_3SO_3H$ | 65 | $C_{15}H_{18}ClN_5O_3S$<br>(383,9) | 18,24<br>17,95 | 251 - 253<br>(Methanol/Ether) |
| 9 | $C_2H_5SO_3H$ | 80 | $C_{16}H_{20}ClN_5O_3S$<br>(397,9) | 17,60<br>17,46 | 200 - 204<br>(Ethanol/Ether) |
| 10 | $HOCH_2CH_2SO_3H$ | 70 | $C_{16}H_{20}ClN_5O_4S$<br>(431,9) | 16,92<br>17,11 | 160 - 163<br>(Ethanol/Ether) |

Beispiel 22

(Z)-2-Amino-5-chlorbenzophenonamidinohydrazonacetat

0,1 Mol (Z)-2-Amino-5-chlorbenzophenonamidinohydrazondihydrochlorid und 0,2 Mol Natriumacetat (wasserfrei) werden mit 500 ml Ethanol übergossen und die Mischung 2h auf dem Wasserbad erwärmt. Nach dem Abkühlen des Ansatzes wird filtriert, mit Ether bis zur Trübung versetzt und erneut filtriert. Danach wird das Filtrat weitgehend eingeengt und der verbleibende,sirupöse Rückstand unter gelindem Erwärmen in Aceton aufgenommen. Beim Anreiben beginnt die Kristallisation. Eine zweite Fraktion kristallisiert aus der Mutterlauge aus, wenn nach Absaugen der Hauptmenge mit wenig Aceton nachgewaschen wird. Ausbeute: 70%

Beispiel 23

(Z)-2-Amino-5-chlorbenzophenonamidinohydrazonacetat

250 mg (E)-2-Amino-5-chlorbenzophenonamidinohydrazon werden in 5 ml Methanol bzw. Aceton gelöst und die Lösung in einem verschlossenen ml-Maßkölbchen in einer Helios-Bestrahlungsapparatur mit zwei HQV-125-Lampen bestrahlt.
Der Fortgang der fotochemischen Reaktion wird UV-spektroskopisch und dünnschichtchromatografisch (Laufmittel: Butylacetat / $CHCl_3$ / wasserfreie Ameisensäure; 3/1/1;v/v/v; Detektion: 1%ige Lösung von 4-Dimethylaminobenzaldehyd in 5 ml konz.Salzsäure und 95 ml Ethanol; Sorbens: Kieselgel G) verfolgt.
Nach 5h ist die Isomerisierung weitgehend abgeschlossen (Gehalt an Ausgangsverbindung: 5%). Die Lösung wird dann i.Vak. bis fast zur Trockne eingeengt, der Rückstand in wenig Aceton aufgenommen und die Lösung mit der äquimolaren Menge Essigsäure versetzt. Die ausgeschiedenen Kristalle werden abgesaugt, mit wenig Aceton gewaschen und getrocknet. Ausbeute: 90%

Beispiel 24

(Z)-2-Amino-5-chlorbenzophenonamidinohydrazonacetat Eine 1%ige Lösung des (E)-2-Amino-5-chlorbenzophenonamidinohydrazons in Aceton wird in einer kreisrunden Durchflußküvette (F = 4,5 $cm^2$, Schichtdicke 0,2 cm) mit einer XBO-500 unter Verwendung des Glasfilters WG9 bestrahlt. Die Durchflußküvette wird im Bestrahlungsstand so angeordnet, daß sie eben voll ausgeleuchtet wird. Bei einmaligem Durchlauf von 10ml/h werden 100 mg/h der Ausgangsverbindung isomerisiert (dc Kontrolle und Aufarbeitung analog Beispiel 23). Ausbeute: 90%

Beispiel 25

(Z)-2-Amino-5-chlorbenzophenonamidinohydrazonacetat

800 mg eines Gemisches von (E)- und (Z)-2-Amino-5-chlorbenzophenonamidinohydrazon (dargestellt durch Behandlung des nach Beispiel 9 erhaltenen Dihydrochloridgemisches analog Beispiel 13) werden mit 100 ml Ethanol versetzt, gerührt und filtriert. Danach wird die Lösung mit einer XBO-500-Lampe unter Verwendung des Glasfilters WG 9 unter ständigem Rühren 6h bestrahlt (dc Kontrolle und Aufarbeitung analog Beispiel 23). Ausbeute: 85%

Beispiel 26

(Z)-2-Amino-5-chlorbenzophenonamidinohydrazonacetat

800 mg (E)-2-Amino-5-chlorbenzophenonamidinohydrazonacetat werden in 100 ml Methanol gelöst und die Lösung mit einer XBO-500-Lampe unter Verwendung des Glasfilters WG 9 unter ständigem Rühren 9h bestrahlt. Der Verlauf der Fotoreaktion wird dc verfolgt (vgl. Beispiel 23). Danach wird die Lösung i.Vak. eingeengt und der Rückstand unter Zusatz von wenig Essigsäure zur Kristallisation gebracht. Ausbeute: 90%

Beispiel 27

(E)-2-Amino-5-chlorbenzophenonamidinohydrazondihydrochlorid

0,1 Mol 2-Amino-5-chlorbenzophenon und 0,027 Mol N,N′-Bis-(2-benzoyl-4-chlorphenyl)-diaminomethan (Walker, G.N., A.R. Engle und R.J.Kempton, J. org. Chemistry 37, 3755 (1972) ) werden mit 0,18 Mol Aminoguanidinhydrochlorid in einem Gemisch von 59 ml Propan-2-ol, 74 ml konz.Salzsäure und 31 ml Wasser innrhalb von 12 min auf eine Innentemperatur von 90 °C erhitzt. Bei beginnendem Rückfluß wird die Heizquelle entfernt und auf 65 °C abgekühlt. Danach wird mit (E)-2-Amino-5-chlorbenzophenonamidinohydrazondihydrochlorid angeimpft, wodurch zwischen 62 und 55 °C die Kristallisation einsetzt.

Unter Luftkühlung läß man auf 45 °C abkühlen, saugt sofort ab, wäscht mit einer Lösung aus 30 ml Propan-2-ol, 37 ml konz.Salzsäure und 15 ml Wasser, danach mit 21 ml Aceton und trocknet bei 50 °C bis zur Gewichtskonstanz. Ausbeute 76%

Beispiel 28

(E)-2-Amino-5-chlorbenzophenonamidinohydrazondihydrochlorid

0,1 Mol 2-Amino-5-chlorbenzophenon, 0,005 Mol N,N′-Bis-(2-benzoyl-4-chlorphenyl)-diaminomethan und 0,12 Mol Aminoguanidinhydrochlorid werden in einem Gemisch aus 62 ml Propan-2-ol, 80 ml konz. Salzsäure und 33 ml Wasser 3,5 h bei 20-25 °C gerührt. Das zunächst orange gefärbte Gemisch hellt sich nach 30-60 min auf, wobei die Kristallisation beginnt. Nach Abschluß des Rührens wird abgesaugt und analog Beispiel 27 weiterbehandelt. Ausbeute: 91%

Beispiel 29

(E)-2-Amino-5-chlorbenzophenonamidinohydrazondihydrochlorid

Ein Gemisch aus 0,1 Mol 2-Amino-5-chlorbenzophenon, 0,008 Mol N,N′-Bis-(2-benzoyl-4-chlorphenyl)-diaminomethan, 0,15 Mol Aminoguanidinhydrochlorid, 65 ml Propan-2-ol, 83 ml konz. Salzsäure und 35 ml Wasser wird in einem Kolben 2 min geschüttelt und danach 68h bei Raumtemperatur stehengelassen.

Die abgeschiedenen Kristalle werden abgesaugt und analog Beispiel 27 weiterbehandelt. Ausbeute: 83%

Beispiel 30

(E)-2-Amino-5-chlorbenzophenonamidinohydrazonhydrochlorid

0,01 Mol 2-Amino-5-chlorbenzophenon und 0,012 Mol Aminoguanidinhydrochlorid werden in einem Gemisch aus 5,7 ml Propan-2-ol, 7,2 ml konz. Salzsäure und 3,0 ml Wasser nach Zugabe von 1,0 ml 0,7-molarer, wäßriger Formaldehydlösung 2,5h bei Raumtemperatur gerührt. Die rötlich-orange Reaktionssuspension wird nach 25-stündigem Stehen bei Raumtemperatur abgesaugt, mit einem Gemisch und 1,0 ml Wasser und anschließend mit ml Ether gewaschen. Das lufttrockene, weiße Produkt wird bei 50 °C getrocknet. Ausbeute: 80%

Beispiel 31

(E)-2-Amino-5-chlorbenzophenonamidinohydrazondihydrochlorid

0,1 Mol 2-Amino-5-chlorbenzophenon und 0,12 Mol Aminoguanidinhydrochlorid werden mit 57 ml Propan-2-ol, 72 ml konz.Salzsäure, 30 ml Wasser sowie 5,2 ml 2%ige Formaldehydlösung versetzt und der orange Ansatz analog Beispiel 30 weiterbehandelt. Ausbeute: 87%

Beispiel 32

(E)-2-Amino-5-chlorbenzophenonamidinohydrazonacetat

0,5 Mol (E)-2-Amino-5-chlorbenzophenonamidinohydrazon werden unter gelindem Erwärmen in 450 ml Aceton gelöst. Die ggf. filtrierte Lösung wird mit 0,5 Mol Essigsäure versetzt, wobei das Acetat sofort

auskristallisiert. Es wird abgesaugt, mit wenig Aceton gewaschen und getrocknet. Ausbeute: 95% Gelbe Kristalle.
Schmb.: ab 186° C u. Zers. (Tröpfchen ab 163° C) (Aceton)
$C_{16}H_{15}ClN_5O_2$ (347,8) Ber.: N 20,13 Gef.: N 20,22
UV [$\lambda_{max}$(nm) (lg$\epsilon$) (Methanol)]; 232(4,39); 253(4,39); 281,5(4,06) (sh); 371(3,78)

Analog können erhalten werden:
(E)-2-Amino-5-chlorbenzophenon-N-phenylamidinohydrazonacetat
Ausbeute: 60% Hellgelbe Nadeln Schmb. 98-107° C (Ethanol/Eisessig)
$C_{22}H_{22}ClN_5O_2$ (423,9) Ber.: N 16,42 Gef.: N 16,75
UV [$\lambda_{max}$(nm) (lg$\epsilon$) (Methanol)]; 233(4,40); 257(4,37); 289(4,17); 373(3,93)
(E)-2-Amino-5-chlorbenzophenon-N-phenylamidinohydrazonhydrochlorid
Ausbeute: 78% Farblose Nadeln Schmb.: 168-174° C (Ethanol/Ether)
UV [$\lambda_{max}$(nm) (lg$\epsilon$) (Methanol)]; 231(4,37); 254(4,38); 287(4,16); 375(3,68)

Analog können weitere (E)-2-Amino-5-chlorbenzophenonamidinohydrazonsalze erhalten werden (s.Tabelle 5).
Sie kristallisieren entweder beim Abkühlen der Lösung oder nach Zusatz von Ether und Anreiben aus.Es wird abgesaugt und umgefällt bzw. umkristallisiert.

TABELLE 5

weitere (E)-2-Amino-5-chlorbenzophenonamidinohydrazonsalze

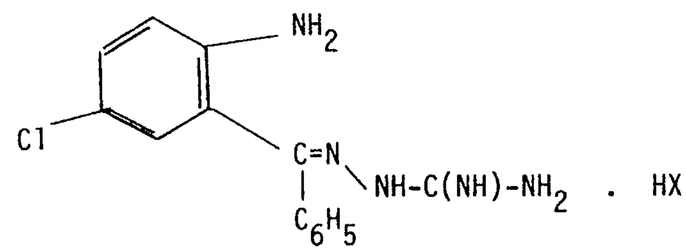

| Nr | HX | Ausb. (%) (bez.a.Base) | Summenformel (Molmasse) | N ber. N gef. | Schmb. °C (Umkrist.) |
|---|---|---|---|---|---|
| 1 | HCOOH | 18 | $C_{15}H_{16}ClN_5O_2$ (333,8) | 20,98 20,71 | 200 - 204 (Propan-1-ol) |
| 2 | $C_2H_5COOH$ | 17 | $C_{17}H_{20}ClN_5O_2$ (361,8) | 19,36 19,45 | 70 - 75 (Methanol) |
| 3 | $C_3H_7COOH$ | 25 | $C_{18}H_{22}ClN_5O_2$ (375,9) | 18,63 18,60 | 102 - 104 135 - 140 (Aceton) |
| 4 | $C_4H_9COOH$ | 45 | $C_{19}H_{24}ClN_5O_2$ (389,9) | 17,96 18,23 | 98 - 110 133 - 152 |
| 5 | $HO-CH_2COOH$ | 58 | $C_{16}H_{18}ClN_5O_3$ (363,8) | 19,25 | 223 - 232 |

Beispeil 33

| Tablettenrezeptur | |
|---|---|
| (Z)-2-Amino-5-chlorbenzophenonamidinohydrazonacetat | 1,5 g |
| Hewten 40 (mikrokristalline Cellulose) | 7,5 g |
| Magnesiumstearat | 0,4 g |
| Aerosil <R> (hochdisperses Siliciumdioxid) | 0,6 g |

Die angegebenen Mengen sind für 100 Tabletten mit einem Durchmesser von 7 mm gedacht, die durch Direktpressung hergestellt werden. Die Tabletten sind unter Lichtschutz aufzubewahren.

Beispiel 34

| Ampullenrezeptur 1 | |
|---|---|
| (Z)-2-Amino-5-chlorbenzophenonamidinohydrazonacetat | 0,015 g |
| aqua ad injectionem | ad 5,0 ml |

Die angegebenen Mengen sind für eine Ampulle gedacht. Für einen Ansatz ist ein Vielfaches der Wirkstoffmenge im entsprechenden Volumen aqua ad injectionem zu lösen und die Lösung nach Sterilfiltration (Verfahren d des AB2-DDR) in 5-ml-Braunglasampullen abzufüllen. Es ist zusätzlich eine vor Lichteinwirkung schützende Verpackung erforderlich. Die Haltbarkeit der Ampullen beträgt 10 Monate.
Vor der Anwendung ist der gesamte Inhalt der Wirkstoffampulle mit 5,0 ml 10%iger Sorbitolinfusionslösung des AB2-DDR zu vermischen (Isotonisierung).

Beispiel 35

| Ampullenrezeptur 2 | |
|---|---|
| (Z)-2-Amino-5-chlorbenzophenonamidinohydrazonacetat | 0,015 g |
| Natriumpyrosulfit | 0,002 g |
| Chelaplex III | 0,002 g |
| 1,2-Propylenglykol | 2,000 g |
| aqua ad injectionem | ad 5,0 ml |

Die angegebenen Mengen sind für eine Ampulle gedacht Für einen Ansatz ist ein Vielfaches der Festsubstanzen mit der entsprechenden Menge 1,2-Propylenglykol zu versetzen und sofort mit aqua ad injectionem zum erforderlichen Volumen aufzufüllen. Nachdem sich alle Bestandteile gelöst haben, erfolgt Sterilfiltration und Abfüllung in 5-ml-Braunglasampullen. Es ist zusätzlich eine vor Lichteinwirkung schützende Verpackung erforderlich.
Die Haltbarkeit der Ampullen beträgt 2 Jahre.

Beispiel 36

| Trockenampullenrezeptur | |
|---|---|
| (Z)-2-Amino-5-chlorbenzophenamidinohydrazonacetat | 0,015 g |
| Sorbitol (oder Mannitol) | 0,250 g |
| aqua ad injectionem | ad 5,0 ml |

Die angegebenen Mengen sind für eine Trockenampulle gedacht. Für einen Ansatz ist ein Vielfaches der Festsubstanzen in der entsprechenden Menge aqua ad injectionem zu lösen, die Lösung nach Sterilfiltration in 5-ml-Braunglas-Kapsolutfläschchen abzufüllen und der Lyophilisation zu unterwerfen.
Vor der Anwendung ist das Lyophylisat in 5,0 ml aqua ad injectionem zu lösen.
Es ist zusätzlich eine vor Lichteinwirkung schützende Verpackung erforderlich.

Beispiel 37

Aconitinarrhythmie Ratte i.v. und p.o.

Die Untersuchungen am Aconitinmodell wurden mit weiblichen Wistarratten (Körpermasse 150-200 g) in Urethannarkose ausgeführt. Die Prüfstoffe wurden 2 min vor Aconitininjektion (50 µg/kg i.v.) intravenös appliziert. Nach dem Schreiben des Normal-EGK erfolgt 1 min nach Substanzgabe eine erneute EKG-Registrierung, um evtl. Eigenwirkungen der Testverbindungen zu erfassen.
3 min nach Aconitinverabfolgung wurde das EKG für 30 sec fortlaufend registriert.
Die orale Applikation erfolgt 1h vor der i.v.-Injektion des Aconitins. Dieser Zeitwert wurde am EKG aufgezeichnet. Desweiteren erfolgte auch hier die Registrierung des EKG für 30 sec 3 min nach Aconitingabe.
Die jeweils stärksten Veränderungen wurden entsprechend Bonitur benotet.

Aus den grafisch dargestellten Dosis-Wirkungs-Geraden erfolgte die Ermittlung einer $ED_{20}$ in mol/kg, welche die Dosis wiedergibt, bei der durch den antiarrhythmischen Effekt einer Verbindung nur noch vereinzelte Extrasystolen nachweisbar sind. Diese Dosis erlaubt eine vergleichende Betrachtung der Wirkungsstärke verschiedener Substanzen untereinander.

Die Verbindungen der allgemeinen Formel 1 zeigten z.T. eine beträchtliche und dosisabhängige,antiarrhythmische Wirkung nach i.v. und p.o. Verabfolgung, wie auch durch die für einige der in Tabelle 1 aufgeführten Vertreter des Beispiels 9 (z.B. Nr. 2, 4, 6, 7 und 8) anhand der i.v.-$ED_{20}$-Werte belegt werden konnte.

Darüberhinaus erwiesen sich beispielsweise das (E)-/(Z)-2-Amino-5-chlorbenzophenonamidinohydrazonnitrat aus Beispiel 2 mit einer $ED_{20}$ von 9,4 . $10^{-6}$ mol/kg i.v. als gut und das (E)-/(Z)-2-Amino-4-chlorbenzophenon-N-phenylamidinohydrazonnitrat aus Beispiel 3 mit einer $ED_{20}$ von 5,6 . $10^{-7}$ mol/kg i.v. als herausragend wirksam gegenüber den aconitininduzierten Arrhythmien an der Ratte.
Die antiarrhythmische Potenz dieser Stoffklasse soll stellvertretend durch vier weitere Derivate demonstriert werden.
So wurden für (E)-2-Amino-2′,5-dichlorbenzophenonamidinohydrazondihydrochlorid aus Beispiel 12 5,6 . $10^{-6}$ mol/kg, für (Z)-2-Amino-5-chlorbenzophenon-N-phenylamidinohydrazon aus Beispiel 13 1,0 . $10^{-6}$ mol/kg sowie für das Acetat und das Hydrochlorid des (E)-2-Amino-5-chlorbenzophenon-N-phenylamidinohydrazons aus Beispiel 32 3,8 . $10^{-6}$ bzw. 1,6 . $10^{-6}$ mol/kg als i.v.-$ED_{20}$-Werte ermittelt.
Die Stärke der antiarrhythmischen Aktivität der bereits eingangs hervorgehobenen Z- und E-Isomeren des 2-Amino-5-chlorbenzophenonamidinohydrazonacetats - wiedergegeben in den Beispielen 21 und 32 - wird in Tabelle 6 noch einmal gegenüber klinisch eingeführten Antiarrhythmika anhand der ermittelten quantitativ vergleichbaren Parameter am Aconitinmodell nach i.v. und p.o. Applikation veranschaulicht.

TABELLE 6

| antiarrhythmische Aktivität von (Z)- bzw. (E)-2-Amino-5-chlorbenzophenonamidinohydrazonacetat | | |
|---|---|---|
| Verbindung | i.v.-$ED_{20}$(mol/kg) | p.o.-$ED_{20}$(mol(kg) |
| Z-Form | $4{,}0 . 10^{-6}$ | $3{,}5 . 10^{-5}$ |
| E-Form | $1{,}1 . 10^{-5}$ | $7{,}8 . 10^{-4}$ |
| Chinidin | $1{,}4 . 10^{-5}$ | $1{,}6 . 10^{-4}$ |
| Ajmalin | $1{,}0 . 10^{-5}$ | $2{,}7 . 10^{-4}$ |
| Procainamid | $1{,}8 . 10^{-4}$ | |
| Disopyramid | $1{,}2 . 10^{-5}$ | $3{,}2 . 10^{-4}$ |
| Lidocain | | $3{,}5 . 10^{-4}$ |
| Propanolol | $2{,}5 . 10^{-5}$ | |
| Talinolol | $9{,}7 . 10^{-6}$ | $1{,}0 . 10^{-3}$ |
| Etmozin | $7{,}2 . 10^{-6}$ | $1{,}8 . 10^{-4}$ |
| Falirythmin | $2{,}7 . 10^{-6}$ | $4{,}5 . 10^{-4}$ |
| Tachmalcor | $5{,}5 . 10^{-7}$ | $5{,}8 . 10^{-5}$ |

Beispiel 38

g-Strophantinarrhythmie Meerscheinchen i.v.

Die Methode beruht auf der Erzeugung ventrikulärer Arrhythmien durch g-Strophantin, das männlichen Meerschweinchen in Urethannarkose (Körpermasse 300-350 g) in der Dosierung von 30 µg/kg/min über die Vena jugularis infundiert wurde. Bewertungskriterien sind hierbei die am EKG unter g-Strophantin zu beobachtenden ersten Extrasystolen sowie der Eintritt der Asystolie. Die Verzögerung des Auftretens dieser Ereignisse unter Substanzgabe wird berechnet und ist als Maß der antiarrhythmischen Effektivität zu werten.

Auch an diesem Modell entfalteten verschiedene Verbindungen der allgemeinen Formel 1 ihre deutlichen arrhythmiehemmenden Eigenschaften. Beispielsweise waren unter Prämedikation von $6{,}25 . 10^{-7}$ mol/kg (Z)-2-Amino-5-chlorbenzophenonamidinohydrazonacetat (Beispiel 21) i.v. 5 min vor g-Strophantininfusion das Auftreten der ersten Extrasystolen um 28% signifikant verzögert. Das entsprechende E-Isomere (Beispiel 30) induzierte z.B. an diesem Modell seinen Schutz in etwas höherer Dosierung: eine 33%ige Verzögerung des Eintritts der ersten Extrasystolen war nach i.v.-Injektion von $1{,}25 . 10^{-6}$ mol/kg zu beobachten.

Für das in Beispiel 3 beschriebene (E)-/(Z)-2-Amino-5-chlorbenzophenon-N-phenylamidinohydrazonnitrat war ebenfalls eine gute antiarrhythmische Wirkung gegenüber der g-strophantininduzierten Extrasystolie nachweisbar, welche sich nach i.v.-Injektion von $1 . 10^{-6}$ mol/kg zu +19% und von $2{,}10 . 10^{-6}$ mol/kg zu +35% berechnen ließ.

Beispiel 39

Koronararterienokklusion Ratte i.v.

Die Untersuchungen zur Beeinflussung der Arrhythmien in der Frühphase nach Koronarokklusion erfolgten an männlichen Wistarratten (Körpermasse 200-250 g) in Pentobarbitalnarkose. Nach Ligatur der linken Koronararterie wurden die Überlebensrate sowie das Auftreten verschiedener Arrhythmieformen innerhalb 20 min nach Okklusion bewertet.

Für die überlebenden Tiere erfolgten zusätzlich die Bestimmung des Zeitpunktes des Einsetzens der Arrhythmien und deren Dauer in min sowie die Zahl der ventrikulären Extrasystolen.

Das Arrhythmiegeschehen in der Frühphase nach Ligatur wurde von einer Reihe Verbindungen der allgemeinen Formel 1 nach i.v.-Injektion im positiven Sinne beeinflußt.

Beispielsweise wirken (Z)- bzw. (E)-2-Amino-5-chlorbenzophenonamidinohydrazonacetat (Beispiel 21 bzw.

32) dosisabhängig und stark protektiv nach i.v. Gabe (3 min vor Okklusion) von 1 . $10^{-5}$ und 2,1 . $10^{-5}$ mol/kg. Die an der Aconitinarrhythmie gefundene gute antiarrhythmische Wirkung wurde damit an der Okklusion bestätigt, was durch die in Tabelle 7 zusammengestellten Ergebnisse dokumentiert werden soll.

TABELLE 7

antiarrhythmische Wirkung von (Z)- bzw. (E)-2-Amino-5-chlorbenzophenonamidinohydrazonacetat bei Koronararterienokklusion (Ratte)

| Versuchsgruppe | Dosis (mol(kg) | n | Überlebensrate n. 20 min (%) | Normaler Sinusrhythmus (%) | Auftreten von Arrhythmien (%) | | | Tod durch Bradyk. (%) |
|---|---|---|---|---|---|---|---|---|
| | | | | | VF | VT | andere | |
| Kontrolle | - | 27 | 33 | 0 | 59 | 82 | 82 | 30 |
| Z-Isomer | 2 . $10^{-5}$ | 23 | 70 s | 26 s | 8 s | 13 s | 54 ns | 25 ns |
| E-Isomer | 2 . $10^{-5}$ | 18 | 71 s | 46 s | 8 s | 13 s | 54 ns | 19 ns |

(VF = Ventrikuläres Flimmern; VT = Ventrikuläre Tachykardie; Bradyk. = Bradydkardie; s = signifikant; ns = nichtsignifikant)

Auch für das (E)-/(Z)-2-Amino-5-chlorbenzophenon-N-phenylamidinohydrazonnitrat (Beispiel 3) konnte an diesem Modell die sehr gute antiarrhythmische Wirksamkeit belegt werden. 5 . $10^{-7}$ mol/kg. i.v. führten zu einer 80%igen Überlebensrate, die signifikant zur Kontrolle erhöht war; einen normalen Sinusrhythmus wiesen 50% der bei dieser Dosis eingesetzten Tiere auf, das Auftreten verschiedener Arrhythmieformen war in jedem Falle signifikant gegenüber der Kontrolle auf 10% (VF), 30% (VT) bzw. 50% (andere) reduziert.

Beispiel 40

Isoprenalinischämie Ratte i.v. und p.o.

Der Myokardschutz durch verschiedene Verbindungen der allgemeinen Formel 1 wurde nach i.v. und p.o. Applikation der Verbindungen am Modell der Isoprenalinischämie der Ratte geprüft.
Die für diese Untersuchungen verwendete Methode erfaßt die Veränderungen des Glykogengehaltes im Herzgewebe unter Isoprenalin und dessen Beeinflussung durch Testsubstanzen. Isoprenalin wurde dabei intraperitoneal in Dosis von 5 mg/kg verabfolgt.
Die i.v. Injektion der Prüfstoffe erfolgte unmittelbar vor der Isoprenalingabe, die orale Applikation 30 min davor.
Als Versuchstiere wurden männliche Wistarratten (Körpermasse 200-250 g) eingesetzt.
Einige der Verbindungen zeigten eine auffallende myokardprotektive Wirksamkeit.
Beispielsweise konnte für das (Z)-2-Amino-5-chlorbenzophenonamidinohydrazonacetat (Beispiel 21) bei beiden Applikationsarten eine starke und dosisabhängige Hemmung der isoprenalininduzierten Glykogenolyse nachgewiesen werden.
Die nach i.v.- bzw. p.o.-Applikation aus je n = 10 Tieren pro Dosis beobachteten Hemmwerte (Mittelwerte) gegenüber der Isoprenalininwirkung sind in Tabelle 8 aufgeführt.

Tabelle 8

| Hemmung der isoprenalininduzierten Glykogenolyse durch (Z)-2-Amino-5-chlorbenzophenonamidinohydrazonacetat | | | |
|---|---|---|---|
| intravenös | | peroral | |
| Dosis (mol/kg) | x % Hemmung | Dosis (mol/kg) | x % Hemmung |
| $3{,}2 \cdot 10^{-6}$ | 5,9 | $7{,}2 \cdot 10^{-5}$ | 37,2 |
| $4{,}6 \cdot 10^{-6}$ | 26,7 | $1{,}0 \cdot 10^{-4}$ | 25,6 |
| $1{,}0 \cdot 10^{-5}$ | 48,7 | $1{,}9 \cdot 10^{-4}$ | 81,4 |
| $1{,}5 \cdot 10^{-5}$ | 37,6 | $3{,}7 \cdot 10^{-4}$ | 71,4 |
| $3{,}2 \cdot 10^{-5}$ | 45,0 | $5{,}0 \cdot 10^{-4}$ | 85,3 |
| $5{,}0 \cdot 10^{-5}$ | 69,6 | | |

Diese Befunde weisen auf das zusätzliche Vorhandensein einer myokardprotektiven Wirkkomponente von (Z)-2-Amino-5-chlorbenzophenonamidinohydrazonacetat hin.

Beispiel 41

Infarktgrößenbestimmung Ratte i.v.

Die Bestimmung der Herzinfarktgröße ist ein wichtiger Parameter zum Nachweis antiischämischer Wirkqualitäten für eine Substanz.
Die hier angewendete markohistochemische Infarktgrößenbestimmung durch Inkubation mit p-Nitroblautetrazoliumsalz (p-NBT) beruht auf einem Dehydrogenasenachweis, wobei p-NBT als Wasserstoffakzeptor dient und die damit verbundene Reduktion des Salzes zu einer Farbänderung desselben führt. Die Beeinflussung der Größe des ischämischen Areals - die Ischämie wurde durch Ligatur der linken Koronararterie männlicher Wistarratten (Körpermasse 200-250 g) erzeugt - wurde nach i.v. Applikation (3 min vor Okklusion) einiger Verbindungen der allgemeinen Formel 1 untersucht. Die hier erhobenen Befunde spiegeln eindeutig die myokardprotektive Wirkkomponente dieser Verbindungen wieder. Für (Z)-2-Amino-5-chlorbenzophenonamidinohydrazonacetat [A] (Beispiel 21) bzw. das (E)-/(Z)-2-Amino-5-chlorbenzophenon-N-phenylamidinohydrazonnitrat [B] (Beispiel 3) wurden die in Tabelle 9 wiedergegebenen Resultate erhalten.

TABELLE 9

| Hemmung der Infarktgröße durch (Z)-2-Amino-5-chlorbenzophenonamidinohydrazonacetat [ A ] und (E)-/(Z)-2-Amino-5-chlorbenzophenon-N-phenylamidinohydrazonnitrat [ B ] | | | | |
|---|---|---|---|---|
| Versuchsgruppe | Dosis (mol/kg) | n | ischämisches Gewebe $\bar{x}$ % | Hemmung der Infarktgröße $\bar{x}$ % |
| Kontrolle | - | 15 | 35,0 | - |
| A | $2 \cdot 10^{-5}$ | 16 | 15,2 | 56,6 s |
| Kontrolle | - | 10 | 46,1 | - |
| B | $1 \cdot 10^{-7}$ | 10 | 21,0 | 54,4 s |

Beispiel 42

Spasmolytische Aktivität in vitro

Die Beeinflussung der glatten Muskelkontraktilität wurde an verschiedenen Organpräparaten gegenüber different angreifenden Agonisten untersucht.
Für diese Versuche wurden isolierte Organe von Wistarratten (Körpermasse 200-300 g, männlich und weiblich) und Meerschweinchen (Körpermasse 250-450 g, männlich) verwendet.
Die Kontaktionsübertragung erfolgt isotonisch über Meßwertgeber (Instrumentenpotentiometer) auf Schreiber.
Aus den durch die Prüfungen der Konzentrations-Wirkungs-Abhängigkeit erhaltenen Ergebnisse wurden mit Hilfe der linearen Regressionsanalyse die $ED_{50}$-Werte in mol/l berechnet.
Die Verbindungen der allgemeinen Formel 1 wiesen deutliche und konzentrationsabhängig auftretende spasmolytische Eigenschaften an differenten glattmuskulären Organpräparaten auf, was als weiterer günstiger Zusatzbefund in dieser Stoffklasse gewertet werden kann. Die hier getroffene Aussage wird durch die in Tabelle 10 wiedergegebene $ED_{50}$Werte für das Beispiel (Z)-2-Amino-5-chlorbenzophenonamidinohydrazonacetat belegt.

TABELLE 10

| spasmolytische Aktivität von (Z)-2-Amino-5-chlorbenzophenonamidinohydrazonacetat | | |
|---|---|---|
| Organpräparat | Antagonismus | $ED_{50}$ (mol(l) |
| Meerschweinchenileum | Histamin | $1{,}1 . 10^{-5}$ |
| Rattenileum | Acetylcholin | $3{,}2 . 10^{-5}$ |
| Rattenileum | Bariumchlorid | $6{,}0 . 10^{-5}$ |
| Fundusstreifen/Rattenmagen | Serotonin | $1{,}9 . 10^{-4}$ |
| Rattenuterus | Serotonin | $1{,}2 . 10^{-6}$ |
| Ductus deferens/Meerschweinchen | Noradrenalin | $1{,}7 . 10^{-4}$ |

Aus diesen Daten wird ersichtlich, daß die Substanz zu einer deutlichen Inhibition der Histamin-, Acetylcholin-, Bariumchlorid-, Serotonin- und Noradrenalin-Kontraktionen an unterschiedlichen Organpräparaten führt, was die Schlußfolgerung nahelegt, daß die Verbindung zusätzlich zu ihrer antiarrhythmischen und myokardprotektiven Wirksamkeit eine unspezifische Hemmung der glatten Muskelkontraktilität verursacht.

## Ansprüche

1. Mittel zur Behandlung von Herz-Kreislauf-Erkrankungen, insbesondere von Arrhythmien, gekennzeichnet durch einen Gehalt einer Verbindung der allgemeinen Formel 1 als Wirkstoff, in der

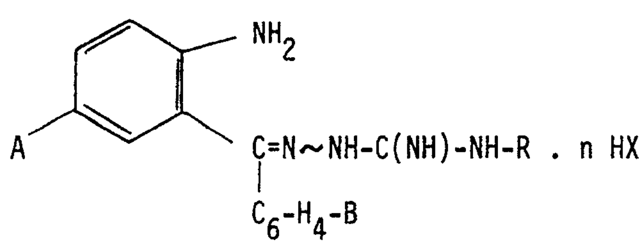

1

A = H, Halogen, $NH_2$ . HX, Alkyl, $NO_2$,
B = H, Halogen, OH, Alkyl,
R = H, Phenyl, subst. Phenyl,
n = 0, 1, 2
HX= eine physiologisch verträgliche Mineralsäure, Alkansulfonsäure, Alkansäure, Alkensäure, Hydroxyalkansäure, Aminoalkansäure, Alkandisäure, Hydroxyalkandisäure bedeuten und die als Z- oder E-Isomere oder als Isomerengemische vorliegen können.
2. Mittel nach Anspruch 1, dadurch gekennzeichnet, daß sie in Form von Tabletten, Dragees, Tropflösungen

oder Injektionslösungen angewendet werden und pro Dosierungseinheit 2 bis 100 mg, vorzugsweise 15 bis mg, einer zu mindestens 90% als Z-Isomeres vorliegenden Verbindung der allgemeinen Formel 1 enthalten.

3. Pharmazeutische Zusammensetzungen, enthaltend neben an sich üblichen pharmazeutischen Hilfsstoffen,wie Trägerstoffen, Gleitmitteln, Isotonisierungszusätzen oder Antioxidantien einen mindestens zu 90% als Z-Isomeres vorliegenden Wirkstoff der allgemeinen Formel 1.

4. Verwendung von Verbindungen der allgemeinen Formel 1 in Form ihrer Z-und/oder E-Isomeren oder der Isomerengemische zur Herstellung von Arzneimitteln zur Behandlung von Herzrhythmusstörungen.

5. Verfahren zur Herstellung von Verbindungen der allgemeinen Formel 1, worin A, B, R sowie n und HX die obige Bedeutung haben, dadurch gekenn zeichnet, daß man

a) ein entsprechend substituiertes 2-Aminobenzophenon der allgemeinen Formel 2, in welcher A und B die o.g. Bedeutung haben, mit einem Hydrazin-Derivat der allgemeinen Formel 3, in welcher D die Bedeutung H, CS-NH-R, CSS-Alkyl oder C(NH)-NH-R sowie R die Bedeutung H, Phenyl oder substituiertes Phenyl haben, in Gegenwart von Mineral- bzw. Sulfonsäuren zu den Zwischenprodukten 4 bzw. 5 umsetzt und diese anschliessend nach bekannten Verfahren in 1 überführt;

b) Zwischenprodukte der allgemeinen Formel 5, worin n = 1 bzw. 2, HY = Mineral- bzw. Sulfonsäuren bedeuten und A, B und R die obige Bedeutung haben, mit Alkalihydroxiden in die Basen der allgemeinen Formel 6 überführt und diese mit Säuren der allgemeinen Formel HX umsetzt bzw. mit Alkalisalzen der Säuren in Alkanolen erhitzt;

c) Zwischenprodukte der allgemeinen Formel 4, worin D = H bedeutet, mit 1-Amidinopyrazolen bzw. Phenylcyanamiden vorzugsweise in Ethanol durch Erhitzen unter Rückflußsieden zu Verbindungen der allgemeinen Formel 6 umsetzt und durch nachfolgende Reaktion mit Säuren der Formel HX in die neuen Verbindungen der Formel 1 überführt;

d) Zwischenprodukte der allgemeinen Formel 4, worin D = CSNH-R bzw. CSS-Alkyl bedeutet, mit Iodalkanen, vorzugsweise in cyclischen Ethern bei Raumtemperatur umsetzt und durch Erhitzen mit Ammoniak Verbindungen der allgemeinen Formel 5 erhält, worin A, B und R die obige Bedeutung haben und HY = HI ist, aus denen durch Überführung in die Basen der allgemeinen Formel 6 und deren Reaktion mit Säuren der allgemeinen Formel HX die neuen Verbindungen 1 hergestellt werden.

6. Verfahren zur Herstellung von Isomeren der allgemeinen Formel (E)-1, in der A, B, R sowie n und HX die obige Bedeutung haben, die dadurch gekennzeichnet sind, daß man die Kondensation der Verbindungen 2 mit Aminoguanidinhydrochlorid (3, D = $C(NH)-NH_2$ . HCl) analog 5a) in einem Gemisch von Propan-2-ol und konz.Salzsäure bei Gegenwart von Formaldehyd oder N,N'-Bis-(2-benzoyl-4-chlorphenyl)-diaminomethan bei Raumtemperatur oder durch kurzzeitiges Erhitzen durchführt.

7. Verfahren zur Herstellung von Isomeren der allgemeinen Formel (Z)-1, in der A, B, R sowie n und HX die obige Bedeutung haben, die dadurch gekennzeichnet sind, daß man

a) Isomerengemische der allgemeinen Formel 5, in der A und B die obige Bedeutung haben und HY die als Katalysator benutzte Mineral- bzw. Sulfonsäure oder HI bedeutet, durch fraktionierte Kristallisation bzw. Umfällung aus niederen Alkoholen in die E- und Z-Isomeren aufStrennt und letztere gemäß 5b) in die Z-Isomeren der neuen Verbindungen 1 überführt;

b) E-Isomere oder Isomerengemische der allgemeinen Formel 5 in einem aprotischen Lösungsmittel unter Zusatz von starken Säuren erhitzt und die erhaltenen Verbindungen (Z)-5 gemäß 5b) in die Verbindungen (Z)-1 überführt;

c) E-Isomere oder Isomerengemische der allgemeinen Formel 5 in einem inerten Lösungsmittel mit Acetanhydrid umsetzt und die entstandenen (Z)-2-Acetaminobenzophenonamidinohydrazone der allgemeinen Formel 7, in der A, B und R die obige Bedeutung haben, in einem Alkanol-Salzsäure-Gemisch entacetyliert werden und die erhaltenen Verbindungen (Z)-5 gemäß 5b) in die Z-Isomeren der neuen Verbindungen 1 überführt;

d) E-Isomere der allgemeinen Formeln 1, 5 bzw. 6 in Ethanol oder Aceton in Konzentrationen von 1 bis 10% löst und mit Licht der Wellenlänge eines für sie typischen, vorzugsweise zwischen 360 und 380 nm liegenden Absorptionsmaximums bestrahlt und sofort die Verbindungen (Z)-1 erhält oder die erhaltenen Verbindungen (Z)-5 bzw. (Z)-6 gemäß 5b) in die Verbindungen (Z)-1 überführt.

8. (E)-/(Z)-2-Aminobenzophenonamidinohydrazondihydrochlorid

9. (E)-/(Z)-2-Aminobenzophenon-N-phenylamidinohydrazondihydrochlorid

10. (E)-2-Amino-5-chlorbenzophenonamidinohydrazon

11. (Z)-2-Amino-5-chlorbenzophenonamidinohydrazon

12. (E)-2-Amino-5-chlorbenzophenonamidinohydrazondihydrochlorid

13. (Z)-2-Amino-5-chlorbenzophenonamidinohydrazondihydrochlorid

14. (E)-2-Amino-5-chlorbenzophenonamidinohydrazonmethansulfonat

15. (Z)-2-Amino-5-chlorbenzophenonamidinohydrazonmethansulfonat

16. (E)-2-Amino-5-chlorbenzophenonamidinohydrazonhydroxyethansulfonat
17. (Z)-2-Amino-5-chlorbenzophenonamidinohydrazonhydroxyethansulfonat
18. (E)-2-Amino-5-chlorbenzophenonamidinohydrazonformiat
19. (Z)-2-Amino-5-chlorbenzophenonamidinohydrazonacetat
20. (E)-2-Amino-5-chlorbenzophenonamidinohydrazonacetat
21. (Z)-2-Amino-5-chlorbenzophenonamidinohydrazonpropionat
22. (E)-2-Amino-5-chlorbenzophenonamidinohydrazonpropionat
23. (Z)-2-Amino-5-chlorbenzophenonamidinohydrazonbutyrat
24. (E)-2-Amino-5-chlorbenzophenonamidinohydrazonbutyrat
25. (Z)-2-Amino-5-chlorbenzophenonamidinohydrazonvalerat
26. (E)-2-Amino-5-chlorbenzophenonamidinohydrazonvalerat
27. (Z)-2-Amino-5-chlorbenzophenonamidinohydrazonmaleat
28. (E)-2-Amino-5-chlorbenzophenonamidinohydrazonhydroxyacetat
29. (Z)-2-Amino-5-chlorbenzophenonamidinohydrazontartrat
30. (Z)-2-Amino-5-chlorbenzophenonamidinohydrazoncitrat
31. (Z)-2-Amino-5-chlorbenzophenon-N-phenylamidinohydrazon
32. (Z)-2-Amino-5-chlorbenzophenon-N-phenylamidinohydrazonacetat
33. (E)-2-Amino-5-chlorbenzophenon-N-phenylamidinohydrazonacetat
34. (E)-2-Amino-5-chlorbenzophenon-N-phenylamidinohydrazonhydrochlorid
35. (E)-/(Z)-2,5-Diaminobenzophenonamidinohydrazontrihydrochlorid
36. (E)-/(Z)-2-Amino-5-brombenzophenonamidinohydrazondihydrochlorid
37. (E)-2-Amino-2′,5-dichlorbenzophenonamidinohydrazondihydrochlorid
38. (Z)-2-Amino-2′,5-dichlorbenzophenonamidinohydrazondihydrochlorid
39. (E)-/(Z)-2-Amino-5-chlor-4′-hydroxybenzophenonamidinohydrazondihydrochlorid
40. (E)-/(Z)-2-Amino-3′-chlorbenzophenonamidinohydrazonhydrochlorid
41. (E)-/(Z)-2-Amino-4′-chlorbenzophenonamidinohydrazondihydrochlorid
42. (E)-/(Z)-2-Amino-4′-methylbenzophenonamidinohydrazondihydrochlorid
43. (E)-2-Amino-5-nitrobenzophenonamidinohydrazonhydrochlorid
44. (E)-/(Z)-2-Amino-2′-chlor-5-nitrobenzophenonamidinohydrazondihydrochlorid.

Europäisches Patentamt

# EUROPÄISCHER RECHERCHENBERICHT

Nummer der Anmeldung

EP 89 89 0328

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | PATENT ABSTRACTS OF JAPAN, Band 11, Nr. 30 (C-400)[2477], 29. Januar 1987; & JP-A-61 200 957 (DAICEL CHEM. IND. LTD) 05-09-1986<br>* Das ganze Dokument *<br>--- | 1 | C 07 C 281/18<br>A 61 K 31/155 |
| D,A | FR-M- 7 858 (UGINE KUHLMANN)<br>* Anspruch 1 *<br>--- | 1 | |
| A | CHEMICAL ABSTRACTS, Band 100, Nr. 17, 23. April 1984, Seite 612, Zusammenfassung Nr. 138714a, Columbus, Ohio, US; J. ZHONG et al.: "Syntheses of antimalarial drugs, derivatives of substituted benzophenones", & YIYAO GONYE 1983, (8), 10-12<br>* Zusammenfassung *<br>----- | 1 | |
| | | | RECHERCHIERTE SACHGEBIETE (Int. Cl.5) |
| | | | C 07 C 281/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 07-08-1990 | PAUWELS G.R.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)